# EUROPÄISCHE PATENTSCHRIFT

(11) **EP 2 812 440 B1**
(45) Veröffentlichungstag und Bekanntmachung des Hinweises auf die Patenterteilung: **26.06.2019**
(21) Anmeldenummer: 13703569.7
(22) Anmeldetag: 06.02.2013
(51) Int. Cl.: C12P 19/02, C12P 19/36, C12P 33/00, C12P 41/00, C13K 11/00, C07D 307/50

(54) **VERFAHREN ZUR HERSTELLUNG VON FURANDERIVATEN AUS GLUCOSE**
METHOD FOR PRODUCTION OF FURANE DERIVATIVES FROM GLUCOSE
PROCÉDÉ POUR LA PRODUCTION DE DÉRIVÉS DE FURANE À PARTIR DU GLUCOSE

(30) Priorität: 07.02.2012 EP 12450007; 03.09.2012 EP 12182758; 12.09.2012 WO PCT/EP2012/067781; 10.12.2012 AT 12842012
(43) Veröffentlichungstag der Anmeldung: 17.12.2014
(73) Patentinhaber: Annikki GmbH, 8074 Raaba-Grambach (AT); Novolana GmbH, 8144 Tobelbad (AT)
(72) Erfinder: ERTL, Ortwin, 8076 Vasoldsberg (AT); STAUNIG, Nicole, 8076 Vasoldsberg (AT); SUT-VEJDA, Marta, A-8010 Graz (AT); MAYER, Bernd, 88453 Erolzheim (DE); MIHOVILOVIC, Marko, A-2380 Perchtoldsdorf (AT); SCHÖN, Michael, A-1140 Wien (AT); HÖLBLING, Johanna, A-4880 St. Georgen/Attergau (AT)
(74) Vertreter: Schwarz & Partner Patentanwälte OG
(86) Internationale Anmeldenummer: PCT/EP2013/052316
(87) Internationale Veröffentlichungsnummer: WO 2013/117585

(56) Entgegenhaltungen:
- WO-A1-86/04353
- WO-A1-2011/124639
- WO-A1-2012/015616
- WO-A2-2009/121785
- CONSTANCE V. VOSS ET AL: "Orchestration of Concurrent Oxidation and Reduction Cycles for Stereoinversion and Deracemisation of sec -Alcohols", JOURNAL OF THE AMERICAN CHEMICAL SOCIETY, Bd. 130, Nr. 42, 22. Oktober 2008 (2008-10-22), Seiten 13969-13972, XP055033012, ISSN: 0002-7863, DOI: 10.1021/ja804816a
- RYAN WOODYER ET AL: "Mechanistic investigation of a highly active phosphite dehydrogenase mutant and its application for NADPH regeneration", FEBS JOURNAL, Bd. 272, Nr. 15, 1. August 2005 (2005-08-01), Seiten 3816-3827, XP055033097, ISSN: 1742-464X, DOI: 10.1111/j.1742-4658.2005.04788.x in der Anmeldung erwähnt
- DANIELA MONTI ET AL: "One-pot multienzymatic synthesis of 12-ketoursodeoxycholic acid: subtle cofactor specificities rule the reaction equilibria of five biocatalysts working in a row", ADVANCED SYNTHESIS & CATALYSIS, WILEY VCH VERLAG, WEINHEIM, DE, Bd. 351, Nr. 9, 1. Juni 2009 (2009-06-01), Seiten 1303-1311, XP002666468, ISSN: 1615-4150, DOI: 10.1002/ADSC.200800727 [gefunden am 2009-03-17] in der Anmeldung erwähnt

## Beschreibung

Die vorliegende Erfindung betrifft ein Verfahren zur Herstellung von Furanderivaten aus D-Glucose in.

Aufgrund steigender Preise für fossile Rohstoffe und einem zu erwarteten Rückgang des Angebots solcher Rohstoffe besteht ein großes Interesse an der Nutzung von nachwachsenden Rohstoffen. Dabei sind die Bereiche Energieerzeugung und Herstellung von Grundchemikalien zu unterscheiden. Die vorliegende Erfindung bezieht sich auf letzteren Bereich und betrifft ein Verfahren zur Herstellung von Furanderivaten aus D-Glucose.

D-Glucose liegt in großer Menge in verschiedensten Biopolymeren vor, welche Bestandteile von nachwachsenden Rohstoffen sind. Beispiele dafür sind Stärke (z.B. Maisstärke) oder Cellulose (z.B. aus lignocellulosischer Biomasse). Zur Herstellung von Furanderivaten ist jedoch Fructose als Ausgangsmaterial wesentlich besser geeignet.

Eine gängige Möglichkeit zur Umwandlung von D-Glucose zu D-Fructose erfolgt unter Verwendung einer geeigneten D-Glucoseisomerase, z.B. D-Xylose-Isomerase, die D-Glucose als Substrat akzeptiert. Solche Verfahren sind schon lange bekannt, z.B. aus US2950228 und auch für den industriellen Einsatz geeignet, wie etwa in US3616221 oder US3868304 beschrieben.

WO 2011/124639 beschreibt ein Verfahren zur Herstellung des Furanderivates Hydroxymethylfurfural ausgehend von Fructose, Glucose oder Mannose, wobei eine Umwandlung von D-Glucose zu D-Fructose mit D-Glucoseisomerase vorgesehen ist.

Ein Problem dabei ist, dass üblicherweise maximal ca. 42 % der D-Glucose zu D-Fructose umgewandelt werden können. Eine weitere Anreicherung von D-Fructose gegenüber D-Glucose kann durch Trennverfahren erreicht werden. Eine Möglichkeit dazu ist die Verwendung von chromatographischen Methoden, wie z.B. in US5221478 beschrieben. Für den Nahrungsmittelsektor wird dabei oft lediglich eine teilweise Anreicherung von D-Fructose angestrebt. Vor allem zur Produktion relativ reiner bis hoch reiner D-Fructose sind chromatographische Verfahren aber sehr aufwändig.

Neben der Verwendung von Isomerasen wurden in der Literatur auch enzymatische Redoxreaktionen an Kohlenhydraten beschrieben.

So wird beispielsweise in DE69839381 eine Sorbitoldehydrogenase beschrieben, die zur Umwandlung von D-Sorbitol zu L-Sorbose eingesetzt werden und bei der Herstellung von Ascorbinsäure Anwendung finden kann.

In DE10247147 ist ein Verfahren beschrieben, bei dem unter Verwendung von D-Mannitol-2-Dehydrogenase D-Fructose zu D-Mannitol reduziert wird.

In US4467033 ist die enzymatische Oxidation von L-Sorbitol zu L-Fructose beschrieben.

Beispiele für die Reduktion von D-Xylose zu Xylitol sind etwa in US20060035353 oder in Woodyer R. et al., FEBS J., 2005, Volume 272, p 3816- 3827 offengelegt.

Es wurde bereits gezeigt, dass geeignete Xylose-Reduktasen verwendet werden können, um D-Glucose zu D-Sorbitol zu reduzieren (z.B. Wang X. et al., Biotechnol. Lett., 2007, Volume 29, p 1409-1412).

Zucker-Redoxenzyme wie z.B. Sorbitol-Dehydrogenase werden auch für diagnostische Zwecke eingesetzt (z.B. DE60006330).

Bei diesen Verfahren handelt es sich um einzelne Redoxreaktionen, bei denen zur Produktbildung jeweils entweder eine Reduktion oder eine Oxidation erfolgt.

Enzymkatalysierte Redoxreaktionen werden in industriellen Prozessen beispielsweise in der Herstellung von chiralen Alkoholen, α-Aminosäuren und α-Hydroxysäuren verwendet. Die bisher bekannten industriellen Prozesse verwenden in der Regel ein Redoxenzym zur Produktsynthese, sowie gegebenenfalls ein weiteres Enzym zur Kofaktor-Regenerierung. Davon zu unterscheiden sind Verfahren, bei denen zwei oder mehr an der Produktbildung beteiligte enzymatische Redoxreaktionen sowie die gegebenenfalls notwendigen enzymatischen Reaktionen zur Kofaktor-Regenerierung (zeitgleich oder sequentiell) in einem Reaktionsansatz durchgeführt werden, ohne dass ein Zwischenprodukt isoliert wird. In letzter Zeit haben solche enzymatischen Kaskadenreaktionen - hier als Ein-Topf-Reaktionen bezeichnet - signifikante Aufmerksamkeit erlangt, da sie effektiv Betriebskosten, Betriebszeit und Umweltauswirkungen reduzieren. Zusätzlich ermöglichen enzymatische Kaskaden von Redoxreaktionen Transformationen, die durch klassische chemische Verfahren nicht einfach umzusetzen sind.

So wurde ein Versuch beschrieben, die Deracemisierung von Racematen sekundärer Alkohole über ein prochirales Keton als Zwischenprodukt unter Verwendung eines Ein-Topf-Systems durchzuführen (J. Am. Chem. Soc., 2008, Volume 130, p. 13969-13972). Die Deracemisierung von sekundären Alkoholen wurde über zwei Alkoholdehydrogenasen (S- und R-spezifisch) mit unterschiedlicher Kofaktor-Spezifizität erreicht. Ein Nachteil des Verfahren ist die sehr geringe Konzentration des eingesetzten Substrats von 0,2-0,5%, was für industrielle Zwecke nicht geeignet ist.

Ein weiteres Ein-Topf-System wurde in WO 2009/121785 beschrieben, wobei ein Stereoisomer eines optisch aktiven sekundären Alkohols zum Keton oxidiert und dann zum entsprechenden optischen Antipoden reduziert wurde und wobei zwei Alkoholdehydrogenasen mit entgegengesetzten Stereoselektivitäten und unterschiedlichen Kofaktor-Spezifitäten verwendet wurden. Die Kofaktoren wurde mittels eines sogenannten "Hydrid-Transfer-Systems" unter Verwendung nur eines zusätzlichen Enzyms regeneriert. Um die Kofaktoren zu regenerieren, wurden verschiedene Enzyme wie z.B. Formatdehydrogenase, Glucosedehydrogenase, Lactatdehydrogenase verwendet. Ein Nachteil dieses Verfahrens ist die geringe Konzentration der verwendeten Substrate.

Im Gegensatz dazu sind bereits viele einzelne enzymatische Redoxreaktionen bekannt. Ein Anwendungsbeispiel ist die Herstellung chiraler Hydroxyverbindungen ausgehend von entsprechenden prochiralen Ketoverbindungen. In diesen Verfahren wird der Kofaktor mittels eines zusätzlichen Enzyms regeneriert. Diesen Verfahren gemeinsam ist, dass sie eine isolierte Reduktionreaktion darstellen und NAD(P)H regenerieren (siehe z.B. EP1152054).

Weitere Beispiele einer enzymatischen Herstellung von chiralen, enantiomerenangereicherten, organischen Verbindungen, wie zum Beispiel Alkoholen oder Aminosäuren, wurden beschrieben (Organic Letters, 2003, Volume 5, p. 3649-3650; US7163815; Biochem. Eng. J., 2008, Volume 39(2) p. 319-327; EP1285962). In den Systemen wurde als Kofaktor-Regenerierungsenzym eine NAD(P)H-abhängige Oxidase aus *Lactobacillus brevis* oder *Lactobacillus sanfranciscensis* verwendet. Bei den Versuchen handelt es sich auch um Einzelreaktionen zur Produktbildung.

Es entfallen bei den genannten einzeln ablaufenden Oxidations- oder Reduktionsreaktionen die Vorteile einer Ein-Topf-Reaktion, wie z.B. Wirtschaftlichkeit durch Zeit- und Materialersparnis.

Eine Isolierung von Fructose aus wässrigen Lösungen ist z.B. gemäß einer Methode, die in US4895601 oder US5047088 beschrieben ist, möglich.

In der Literatur sind verschiedene Beispiele zur Produktion von Furanderivaten aus Kohlenhydraten bekannt.

In solchen Verfahren wurden eine Vielzahl an sauren Katalysatoren verwendet: anorganische Säuren (siehe z. B. Chheda, J. N.; Roman-Leshkow, Y.; Dumesic, J. A. Green Chem. 2007, 9, 342-350), organische Säuren (z. B. Oxalsäure), Zeolithe (H-Form), Übergangsmetallionen (siehe z. B. Young, G.; Zhang, Y.; Ying, J. Y. Angew. Chem. Int. Ed. 2008, 47, 9345-9348; Tyrlik, S. K.; Szerszen, D.; Olejnik, M.; Danikiewicz, W. Carbohydr. Res. 1999, 315, 268-272), heterogen gelöste Metallphosphate (siehe z. B. Asghari, F. S.; Yoshida, H. Carbohydr. Res. 2006, 341, 2379-2387) oder auch stark saure Kationenaustauscher (siehe z.B. Villard, R.; Robert, F.; Blank, I.; Bernardinelli, G.; Soldo, T.; Hofmann, T. J. Agric. Food Chem. 2003, 51, 4040-4045).

Als Lösungsmittel in solchen Prozessen wurde Wasser, als grünes Lösungsmittel, bevorzugt untersucht. Obwohl ein System aus Biomasse und Wasser als "green approach" gewertet werden kann, so kann bei Temperaturen von >300°C und Drücken von über 20 MPa, die benötigt werden um akzeptable Ausbeuten zu erzielen, nicht mehr von einem solchen gesprochen werden (siehe z. B. Qi, X.; Watanabe, M.; Aida, T. M.; Smith Jr., R. S. Cat. Commun. 2008, 9, 2244-2249).

Eine spezielle Furanverbindung die aus Kohlenhydraten in Gegenwart von sauren Katalysatoren hergestellt werden kann, stellt Hydroxymethylfurfural (im Weiteren HMF) dar. Prozesse zur Produktion von HMF sind ebenfalls literaturbekannt. HMF kann in wässriger Lösung in Gegenwart von homogenen und heterogenen Säuren aus Kohlenhydraten gewonnen werden. Die erreichten Ausbeuten hierbei betragen je nach Kohlenhydrat-Substrat und Reaktionsbedingungen zwischen 30 und 60 %. Wird Wasser als Lösungsmittel eingesetzt, so werden auch hier Reaktionsbedingungen von 300 °C und 27 MPa beschrieben. Zudem wird die Bildung von Nebenprodukten wie Lävulinsäure (LS) oder unlöslichen Huminsäuren beschrieben (siehe z.B. Bicker, M., Kaiser, D., Ott, L., Vogel, H., J. of Supercrit. Fluids 2005, 36, 118-126; Szmant, H. H., Chundury, D. D., J. Chem. Techn. Biotechnol. 1981, 31, 135-145; Srokol, Z., Bouche, A.-G., van Estrik, A., Strik, R. C. J., Maschmeyer, T., Peters, J. A., Carbohydr. Res. 2004, 339, 1717-1726).

Ein Durchflussprozess unter superkritischen Bedingungen ausgehend von D-Glucose wurde von Aida et al. beschrieben (Aida, T. A.; Sato, Y.; Watanabe, M.; Tajima, K.; Nonaka, T.; Hattori, H.; Arai, K. J. of Supercrit. Fluids, 2007, 40, 381-388).

Organische Lösungsmittel können bei der HMF-Herstellung ebenfalls geeignet sein. Eine wichtige Einschränkung ist hierbei jedoch, dass sie in manchen Fällen schwer vom Produkt abzutrennen sind (siehe z. B. Bao, Q.; Qiao, K.; Tomido, D.; Yokoyama, C. Catal. Commun. 2008, 9, 1383-1388; Halliday, G. A.; Young Jr., R. J.; Grushin, V. V. Org. Lett. 2003, 5, 2003-2005). Zusätzlich sind viele in der Vergangenheit eingesetzte Lösungsmittel nicht für mögliche Folgereaktionen geeignet, sondern erzeugen Nebenprodukte, sofern sie nicht abgetrennt werden. Häufig eingesetzte Lösungsmittel für die Umsetzung von Kohlenhydraten zu HMF sind Dimethylsulfoxid (DMSO) und Dimethylformamid (DMF). Im Vergleich zu Wasser als Lösungsmittel können die Umsetzungen von Kohlenhydraten zu HMF in diesen Fällen schon bei vergleichsweise niedrigen Temperaturen von 80 - 140 °C durchgeführt werden und liefern deutlich höhere Ausbeuten (bis zu 95 % in DMF) in kürzeren Reaktionszeiten (30 min bis 2 h) (siehe z. B. Halliday, G. A., Young Jr., R. J., Grushin, V. V., Org. Lett. 2003, 5, 2003-2005; WO2009076627. Es wird vermutet, dass DMSO in der Dehydratisierung von D-Fructose (oder anderen Kohlenhydraten) zu HMF (und vergleichbaren Verbindungen) als Katalysator agiert (siehe: Amarasekara, A. S.; Williams, L. D.; Ebede, C. C. Carbohydr. Res. 2008, 343, 3021-3024).

Reaktionsgemische aus Wasser/DMSO oder Wasser/Toluol wurden ebenfalls in einer kontinuierlichen Reaktionsführung eingesetzt, wobei Reaktionszeiten von 4-6 h bei 140-180°C benötigt wurden, um bestenfalls 80 % Ausbeute an HMF zu erhalten (siehe Chheda, J. N., Roman-Leshkov, Y., Dumesic, J. A., Green Chem. 2007, 9, 342-350).

Ionische Flüssigkeiten können sowohl als neutrale Lösungsmittel als auch als aktive Brønsted-Säuren agieren, wobei die Abtrennung der ionischen Flüssigkeiten auch weiterhin ein Problem darstellt. Immobilisierte ionische Flüssigkeiten wurden zudem als Brønsted-Säure-Katalysatoren verwendet (siehe Bao, Q.; Qiao, K.; Tomido, D.; Yokoyama, C. Catal. Commun. 2008, 9, 1383-1388).

Alle bis heute bekannten Prozesse weisen verschiedene Nachteile auf, beispielsweise geringe Anfangskonzentration des Substrates, niedrige Gesamtausbeuten.

Es wurde nun überraschenderweise eine Möglichkeit gefunden, eine bessere Gesamtausbeute bei der Gewinnung von Furanderivaten aus D-Glucose zu erreichen, bei der überraschend hohe Anfangskonzentration der D-Glucose verwendet werden können.

In einem Aspekt stellt die vorliegende Erfindung ein Verfahren zur Gewinnung von Furanderivaten aus D-Glucose zur Verfügung, dass dadurch gekennzeichnet ist, dass
A) D-Glucose in einem enzymatischen Verfahren unter Verwendung und Regenerierung von Redoxkofaktoren in D-Fructose übergeführt wird,
   wobei D-Glucose unter Beteiligung von zwei oder mehr Oxidoreduktasen in D-Fructose übergeführt wird und bei der Umwandlung von D-Glucose zu D-Fructose zuerst eine enzymatisch katalysierte Reduktion zu D-Sorbitol und anschließend eine enzymatisch katalysierte Oxidation von D-Sorbitol zu D-Fructose durchgeführt werden,
   wobei als Redoxkofaktoren NAD⁺/NADH und NADP⁺/NADPH verwendet werden und
   wobei als Resultat mindestens zweier weiterer im selben Reaktionsansatz ablaufender enzymatisch katalysierter Redoxreaktionen einer der beiden Redoxkofaktoren in seiner reduzierten Form anfällt und der jeweils andere in seiner oxidierten Form, und
B) D-Fructose zu Furanderivaten umgesetzt wird und
in Stufe A)
- bei der Regenerierungsreaktion, die den reduzierten Kofaktor wieder in seine ursprüngliche oxidierte Form überführt, Sauerstoff oder eine Verbindung der allgemeinen Formel 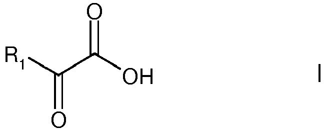 worin R₁ für eine geradkettige oder verzweigtkettige (C₁-C₄)-Alkylgruppe oder für eine (C₁-C₄)-Carboxyalkylgruppe steht, reduziert wird, und
- bei der Regenerierungsreaktion, die den oxidierten Kofaktor wieder in seine ursprüngliche reduzierte Form überführt, ein (C₄-C₈)-Cycloalkanol oder eine Verbindung der allgemeinen Formel 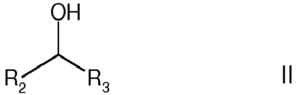 worin R₂ und R₃ unabhängig voneinander ausgewählt sind aus der Gruppe bestehend aus H, (C₁-C₆)-Alkyl, worin Alkyl geradkettig oder verzweigt ist, (C₁-C₆)-Alkenyl, worin Alkenyl geradkettig oder verzweigt ist und ein bis drei Doppelbindungen enthält, Aryl, insbesondere C₆-C₁₂ Aryl, Carboxyl, oder (C₁-C₄)-Carboxyalkyl, insbesondere auch Cycloalkyl, z.B. C₃-C₈ Cycloalkyl, oxidiert wird.

In einem weiteren Aspekt in einem Verfahren gemäß vorliegender Erfindung ist R₁ eine substituierte oder unsubstituierte, z.B. eine unsubstiuierte C1-C4-Alkylgruppe.

In einem weiteren Aspekt sind in einem Verfahren gemäß vorliegender Erfindung R₂ und R₃ unabhängig voneinander ausgewählt aus der Gruppe bestehend aus H, (C₁-C₆)-Alkyl, worin Alkyl geradkettig oder verzweigt ist, (C₁-C₆)-Alkenyl, worin Alkenyl geradkettig oder verzweigt ist und ein bis drei Doppelbindungen enthält, Aryl, insbesondere C₆-C₁₂ Aryl, Carboxyl, oder (C₁-C₄)-Carboxyalkyl.

Ein Verfahren, das durch die vorliegende Erfindung bereitgestellt wird, wird hier auch als Verfahren gemäß/nach vorliegender Erfindung bezeichnet.

Als "Oxidationsreaktion(en)" und "Reduktionsreaktion(en)" werden hier diejenigen enzymkatalysierten Redoxreaktionen bezeichnet, die nicht Teil der Kofaktor-Regenerierung sind und in einem Verfahren gemäß vorliegender Erfindung an der Bildung des Produkts beteiligt sind. "Oxidationsreaktion(en)" und "Reduktionsreaktion(en)" sind unter dem Begriff "Produktbildungsreaktionen" zusammengefasst. Die Produktbildungsreaktionen in einem Verfahren gemäß vorliegender Erfindung beinhalten jeweils mindestens eine Oxidationsreaktion sowie mindestens eine Reduktionsreaktion.
In einer bevorzugten Ausführungsform der vorliegenden Erfindung ist ein Verfahren gemäß vorliegender Erfindung dadurch gekennzeichnet, dass Oxidationsreaktion und Reduktionsreaktion zeitlich parallel ablaufen.

Enzyme und Redoxenzyme in einem Verfahren gemäß vorliegender Erfindung schließen Oxidoreduktasen ein. Oxidoreduktasen sind Enzyme, die Redoxreaktionen katalysieren. Oxidoreduktasen schließen beispielsweise Dehydrogenasen, Reduktasen, Oxidasen, Katalasen ein.

Die Nennung einer Säure oder des Salzes einer Säure schließt hier den jeweils nicht genannten Begriff ein. Ebenfalls schließt die Nennung von Säuren, insbesondere Gallensäuren, hier alle davon abgeleiteten Ester mit ein. Weiter sind hier (partiell) mit Schutzgruppen versehene Verbindungen bei der Nennung der zugrundeliegenden Substanzen mit eingeschlossen.

In einer bevorzugten Ausführungsform der vorliegenden Erfindung ist ein Verfahren gemäß vorliegender Erfindung dadurch gekennzeichnet, dass als Verbindung der Formel I (2-Oxosäure) Pyruvat (Redoxkosubstrat) eingesetzt wird, welches mittels einer Laktatdehydrogenase zu Laktat reduziert wird, das heißt, dass bei der Regenerierungsreaktion, die den reduzierten Kofaktor wieder in seine ursprüngliche oxidierte Form überführt mittels einer Laktatdehydrogenase Pyruvat zu Laktat reduziert wird.

In einer bevorzugten Ausführungsform der vorliegenden Erfindung ist ein Verfahren gemäß vorliegender Erfindung dadurch gekennzeichnet, dass als Verbindung der Formel II (Redoxkosubstrat) ein sekundärer Alkohol, insbesondere 2-Propanol (Isopropylalkohol, IPA) eingesetzt wird, welches mittels einer Alkoholdehydrogenase zu Aceton oxidiert wird, das heißt, dass bei der Regenerierungsreaktion, die den oxidierten Kofaktor wieder in seine ursprüngliche reduzierte Form überführt mittels einer Alkoholdehydrogenase 2-Propanol zu Aceton oxidiert wird.

In einer bevorzugten Ausführungsform der vorliegenden Erfindung ist ein Verfahren gemäß vorliegender Erfindung dadurch gekennzeichnet, dass als Redoxkosubstrat Sauerstoff eingesetzt wird, welcher mittels einer NADH Oxidase reduziert wird.

In einer bevorzugten Ausführungsform der vorliegenden Erfindung ist ein Verfahren gemäß vorliegender Erfindung dadurch gekennzeichnet, dass als Redoxkosubstrat ein sekundärer Alkohol Malat eingesetzt wird, welches mittels einer Oxalacetat-decarboylierenden Malatdehydrogenase ("Malatenzym") zu Pyruvat und CO₂ oxidiert wird, z.B. dass bei der Regenerierungsreaktion, die den oxidierten Kofaktor wieder in seine ursprüngliche reduzierte Form überführt mittels einer Malatdehydrogenase Malat zu Pyruvat und CO₂ oxidiert wird.

Das entstehende Pyruvat wird bei dieser Ausführungsform in einer weiteren Redoxreaktion umgesetzt, die nicht zur Produktbildung dient, sondern die zweite Kofaktor-Regenerierungsreaktion darstellt.

Geeignete Quellen für D-Glucose in einem Verfahren nach vorliegender Erfindung schließen zum Beispiel enzymatische oder nicht-enzymatische Hydrolysate von Stärke, insbesondere Maisstärke, enzymatische oder nicht-enzymatische Hydrolysate von Saccharose oder enzymatische oder nicht-enzymatische Hydrolysate von Cellulose ein. Cellulose, die in einem Verfahren nach vorliegender Erfindung verwendet wird kann zum Beispiel gewonnen werden aus Biomasse, vorzugsweise aus lignocellulosischer Biomasse, wie zum Beispiel Holz, Stroh, wie Weizenstroh, Maisstroh, Bagasse, Sisal, Energiegräser. Zur enzymatischen Hydrolyse von Maisstärke können z. B. Amylasen eingesetzt werden. Für enzymatische Spaltung von Saccharose eignen sich z. B. Invertasen. Zur enzymatischen Spaltung von Cellulose können z. B. Cellulasen eingesetzt werden. Für die nicht-enzymatische Spaltung genannter Mehrfachzucker eignet sich zum Beispiel eine säurekatalysierte Spaltung.

Die Stufe A) in einem Verfahren gemäß vorliegender Erfindung wird in einem wässrigen System, dem gegebenenfalls ein Puffer zugesetzt wird, durchgeführt. Geeignete Puffer schließen zum Beispiel Acetat-, Kaliumphosphat-, Tris-HCl- und Glycin-Puffer zum Beispiel mit einem pH-Wert von 5 bis 10,5, vorzugsweise von 6 bis 10 ein. Weiters, oder alternativ können dem System bei der Umsetzung von D-Glucose zu D-Fructose Ionen zur Stabilisierung der Enzyme, wie zum Beispiel Mg²⁺ oder sonstige Zusätze wie zum Beispiel Glycerin zugesetzt werden.

In einem Verfahren gemäß vorliegender Erfindung wird in Stufe A) D-Glucose in D-Fructose gemäß dem Reaktionsschema 1 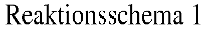 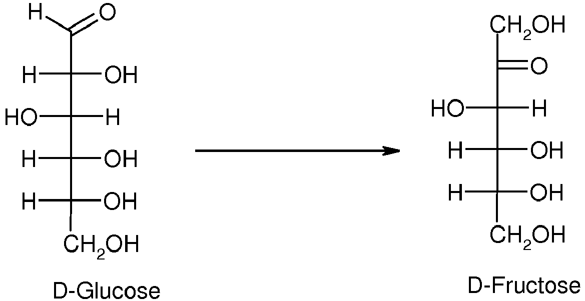 übergeführt.

Die vorliegende Erfindung ist dadurch gekennzeichnet, dass bei der Umwandlung von D-Glucose zu D-Fructose zuerst eine enzymatisch katalysierte Reduktion und anschließend eine enzymatisch katalysierte Oxidation durchgeführt werden. In einem besonderen Aspekt ist die vorliegenden Erfindung dadurch gekennzeichnet, dass eine Isomerisierung der D-Glucose durch Reduktion zu D-Sorbitol erfolgt, das zur D-Fructose oxidiert wird, insbesondere nach dem folgenden Reaktionsschema 2

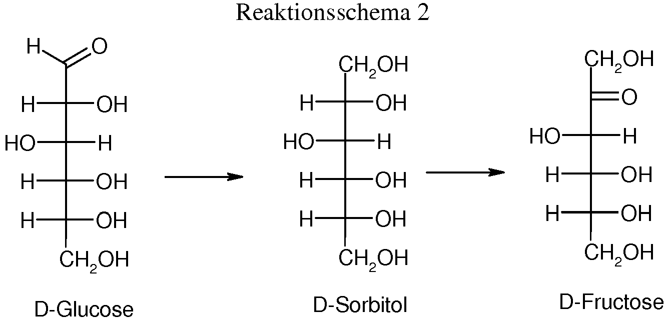

Das Verfahren nach vorliegender Erfindung ist dadurch gekennzeichnet, dass sowohl Reduktions- als auch Oxidationsreaktion(en) zur Umwandlung von D-Glucose zu D-Fructose im selben Reaktionsansatz ohne Isolierung von Zwischenprodukten erfolgen.

Geeignete Enzyme zur Reduktion von D-Glucose zu D-Sorbitol sind bekannt und schließen z.B. Xylosereduktasen ein, die beispielsweise aus *Candida tropicalis* oder aus *Candida parapsilosis* erhältlich sind.

Geeignete Enzyme zur Oxidation von D-Sorbitol zu D-Fructose sind bekannt und schließen z.B. Sorbitoldehydrogenasen ein, die beispielsweise aus Schafsleber, *Bacillus subtilis* oder *Malus domestica* erhältlich sind.

Eine besondere Ausführungsform des Verfahrens gemäß vorliegender Erfindung ist dadurch gekennzeichnet, dass an der Umwandlung von D-Glucose zu D-Fructose mindestens eine Dehydrogenase eingesetzt werden.

Sowohl die Enzyme, als auch die Redoxkofaktoren können dabei entweder in löslicher Form oder an einen Träger (Feststoff) immobilisiert zum Einsatz kommen.

Redoxenzyme, die geeignet sind, NAD⁺/NADH und/oder NADP⁺/NADPH durch zu regenerieren sind bekannt und dem Fachmann geläufig und schließen z.B. Dehydrogenasen ein.

Bei einem Verfahren nach vorliegender Erfindung können in Stufe A) sowohl Einzelenzyme als auch Fusionsproteine umfassend zwei Redoxenzyme zum Einsatz kommen.

Eine weitere, besondere Ausführungsform des Verfahrens nach vorliegender Erfindung ist dadurch gekennzeichnet, dass enzymatische Redoxreaktionen zur Umsetzung von D-Glucose zu D-Fructose durch solche Dehydrogenasen katalysiert werden, welche die Redoxkofaktoren NAD⁺/NADH und/oder NADP⁺/NADPH verwenden.

Dabei bezeichnet NAD⁺ die oxidierte Form und NADH die reduzierte Form von Nicotinamidadenindinucleotid während NADP⁺ die oxidierte Form und NADPH die reduzierte Form von Nicotinamidadenindinucleotidphosphat bezeichnen. Ein Zusatz von Redoxkofaktoren ist u. U. nicht notwendig, wenn die Enzym-Lösungen diese bereits in ausreichender Konzentration enthalten. Falls die Redoxkofaktoren NAD(P)⁺ und/oder NAD(P)H bei der Umsetzung von D-Glucose zu D-Fructose zugesetzt werden, beträgt die zugesetzte Konzentration in einem Verfahren gemäß vorliegender Erfindung üblicherweise von 0,001 mM bis 10 mM, vorzugsweise von 0,01 mM bis 1 mM.

Weitere Redoxenzyme zur Regenerierung der Redoxfaktoren sind dem Fachmann bekannt und schließen z.B. Alkoholdehydrogenasen, NADH-Oxidasen, Hydrogenasen, Laktatdehydrogenasen oder Formiatdehydrogenasen ein.

Eine weitere, besondere Ausführungsform des Verfahrens nach vorliegender Erfindung ist dadurch gekennzeichnet, dass NAD⁺ bei der Umsetzung von D-Glucose zu D-Fructose im selben Reaktionsansatz durch eine NADH-Oxidase regeneriert wird.

Eine weitere, besondere Ausführungsform des Verfahrens nach vorliegender Erfindung ist dadurch gekennzeichnet, dass NADPH bei der Umsetzung von D-Glucose zu D-Fructose im selben Reaktionsansatz durch eine Alkoholdehydrogenase regeneriert wird.

NADH-Oxidasen und Alkoholdehydrogenasen sind dem Fachmann bekannt. Alkoholdehydrogenasen schließen z.B. solche aus *Lactobacillus kefir* ein. Geeignete NADH Oxidasen sind zum Beispiel erhältlich aus *Leuconostoc mesenteroides, Streptococcus mutans, Clostridium aminovalericum.*

Eine weitere, besondere Ausführungsform des Verfahrens nach vorliegender Erfindung ist dadurch gekennzeichnet, dass NADPH bei der Umsetzung von D-Glucose zu D-Fructose im selben Reaktionsansatz durch die Alkoholdehydrogenase aus *Lactobacillus kefir* regeneriert wird.

Zur Regenerierung der Redoxkofaktoren müssen Co-Substrate vorhanden sein und gegebenenfalls zugesetzt werden.

Als Co-Substrate werden Substanzen bezeichnet, die bei der Regenerierung von NAD⁺/NADH und/oder NADP⁺/NADPH (oder anderer Redoxcofaktoren) reduziert oder oxidiert werden. Geeignete Co-Substrate bei einem Verfahren nach vorliegender Erfindung schließen beispielsweise Alkohole (z.B. 2-Propanol), Milchsäure und deren Salze, Brenztraubensäure und deren Salze, Sauerstoff, Wasserstoff und/oder Ameisensäure und deren Salze ein.

NADPH kann beispielsweise durch die Alkoholdehydrogenase aus *Lactobacillus kefir* unter Zusatz des Co-Substrats 2-Propanol (Isopropanol), das zu Aceton oxidiert wird, regeneriert werden.

Mögliche Reaktionswege für die Umwandlung von D-Glucose zu D-Fructose gemäß einem Verfahren nach vorliegender Erfindung sind in den nachfolgenden Reaktionsschemata 3 und 4 gezeigt: 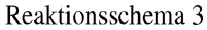 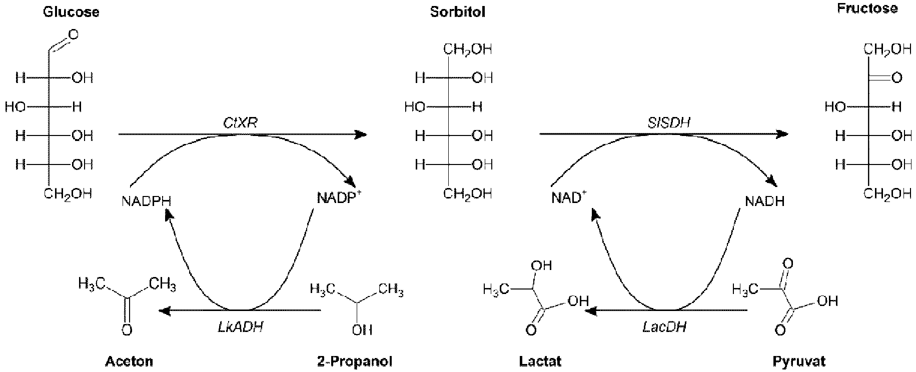
- CtXR: = Xylosereduktase aus *Candida tropicalis*
- SISDH: = Sorbitoldehydrogenase aus Schafsleber
- LkADH: = Alkoholdehydrogenase aus *Lactobacillus kefir,* NADP(H)-abhängig
- LacDH: = Laktatdehydrogenase, NAD(H)-abhängig

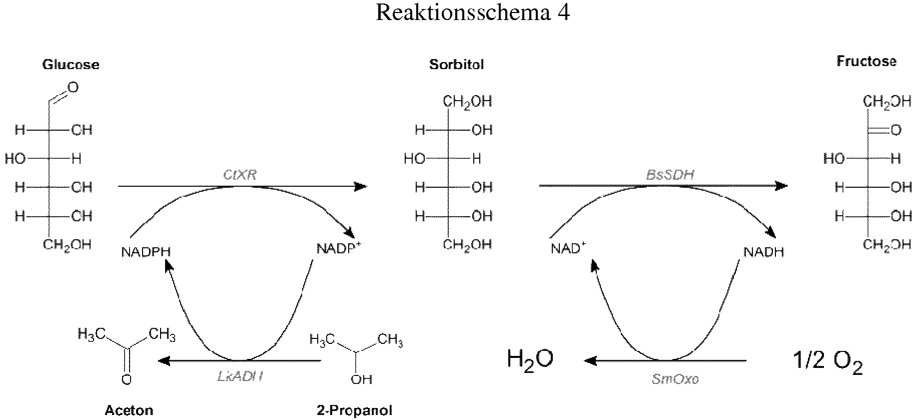

- CtXR: = Xylosereduktase aus *Candida tropicalis*
- BsSDH: = Sorbitol-Dehydrogenase aus *Bacillus subtilis*
- LkADH: = Alkoholdehydrogenase aus *Lactobacillus kefir,* NADP(H)-abhängig
- SmOxo: = NADH Oxidase aus *Streptococcus mutans*

Es wurde gefunden, dass in einem Verfahren gemäß vorliegender Erfindung bei der Umsetzung von D-Glucose zu D-Fructose eine hohe Anfangskonzentration der D-Glucose in der wässrigen Reaktionsmischung von ≥5 % (w/v) D-Glucose, bevorzugt ≥ 10 % (w/v) D-Glucose, insbesondere bevorzugt ≥ 15 % (w/v) D-Glucose eingesetzt werden kann.

In einer weiteren, bevorzugten Ausführungsform wird die D-Glucose in einem Verfahren gemäß vorliegender Erfindung in der wässrigen Reaktionsmischung in einer Konzentration von ≥5 % (w/v) D-Glucose, bevorzugt ≥ 10 % (w/v) D-Glucose, insbesondere bevorzugt ≥ 15 % (w/v) D-Glucose, wobei eine Konzentration von 50% (w/v), vorzugsweise 40% (w/v), insbesondere bevorzugt 35% (w/v) nicht überschritten werden sollte.

Aufgrund der temperaturabhängigen Löslichkeit von D-Glucose ist bei der Durchführung des Verfahrens die Glucose-Konzentration der jeweiligen Reaktionstemperatur anzupassen.

Enzyme können in einem Verfahren gemäß vorliegender Erfindung als solche, gegebenenfalls in der Form von Zell-Lysaten, gegebenenfalls als rekombinant überexprimierte Proteine, beispielsweise als in *E. coli* rekombinant überexprimierte Proteine verwendet werden, wobei weiterhin bevorzugt die entsprechenden Zell-Lysate ohne weitere Aufreinigung eingesetzt werden können. Je nach dem zu produzierenden Enzym können auch andere Mikroorganismen zur Expression eingesetzt werden, z.B. Mikroorganismen, die dem Fachmann bekannt sind. Feste Bestandteile der jeweiligen Mikroorganismen können in einem Verfahren gemäß vorliegender Erfindung entweder abgetrennt werden oder in der Reaktion mit eingesetzt werden (z.B. Ganzzell-Biokatalysatoren). Es können auch Kulturüberstände oder Lysate von Mikroorganismen eingesetzt werden, die ohne rekombinante DNA-Technologie bereits ausreichende Enzym-Aktivitäten aufweisen. In einem Verfahren gemäß vorliegender Erfindung können sowohl Enzyme als auch Redoxkofaktoren entweder in löslicher Form oder an Feststoffe immobilisiert zum Einsatz kommen. Die Enzym-Einheit 1 U entspricht dabei derjenigen Enzymmenge, die benötigt wird, um 1 µmol Substrat pro min umzusetzen.

Überraschenderweise wurde gefunden, dass in einem Verfahren gemäß vorliegender Erfindung bei der Umsetzung von D-Glucose zu D-Fructose ein hoher Umsatz erreicht werden kann, beispielsweise ein Umsatz von ≥70% (w/v), wie ≥90% (w/v), z.B. ≥98% (w/v) und bis zu 99,9% (w/v), oder gar ein vollständiger Umsatz erreicht werden kann.

Abhängig von den verwendeten Enzymen kann das Verfahren nach vorliegender Erfindung, beispielsweise in Stufe A), bei Temperaturen von 10°C bis 70°C, vorzugsweise von Raumtemperatur, z.B. 20°C, bis 50°C durchgeführt werden.

Die D-Fructose, die gemäß Stufe A) der vorliegenden Erfindung gewonnen werden kann, kann, z.B. mit Hilfe einer Kristallisation, isoliert werden.

Der beispielsweise bei der Spaltung von Saccharose anfallende 50-%ige D-Glucose-Anteil kann mit einem zweistufigen, enzymatischen Redoxverfahren nach vorliegender Erfindung zu D-Fructose umgewandelt werden, wobei es zu einer Erhöhung des Anteils von D-Fructose am Gesamt-Zuckergehalt kommt. Damit ist ein geeignetes Ausgangsmaterial zur weiteren Umwandlung in Furanderivate zugänglich, wobei überraschenderweise gefunden wurde, dass sich das Zwischenprodukt D-Fructose, das nach einem Verfahren gemäß vorliegender Erfindung erhalten wird, besonders gut für die weitere Umwandlung zu Furanderivaten eingesetzt werden kann.

Die Umsetzung der D-Fructose zu Furanderivaten in Stufe B) gemäß vorliegender Erfindung kann nach einer geeigneten Methode erfolgen, z.B. nach einer üblichen Methode, oder wie hierin beschrieben.

Gemäß üblicher Methoden kann die Umsetzung der D-Fructose zu Furanderivaten in einem Verfahren gemäß vorliegender Erfindung in Gegenwart eines Katalysators, z.B. eines sauren Katalysators, wie einer anorganischen Säure, organische Säure, z. B. Oxalsäure, eines Zeolith (H-Form), von Übergangsmetallionen, eines heterogen gelösten Metallphosphats, eines stark sauren Kationenaustauscher, erfolgen.

Als Lösungsmittel in solchen Prozessen kann Wasser oder ein organisches Lösungsmittel Verwendung finden, z.B. Dimethylsulfoxid (DMSO), Dimethylformamid (DMF), N-Methylpyrrolidon.

Bevorzugt erfolgt die Umsetzung der D-Fructose zu Furanderivaten in Stufe B) gemäß vorliegender Erfindung in Gegenwart eines sauren Katalysators und in Gegenwart von N-Methyl-pyrrolidon (N-Methyl-2-pyrrolidon, NMP) der Formel

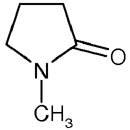

Die Umsetzung der D-Fructose zu Furanderivaten in Stufe B) gemäß vorliegender Erfindung kann entweder als Batch-Prozess oder als kontinuierlicher Prozess durchgeführt werden.

In einer bevorzugten Ausführungsform wird die Stufe B) gemäß vorliegender Erfindung unter Mikrowellen-Erhitzung durchgeführt.

Besondere Ausführungsformen des Verfahrens nach vorliegender Erfindung sind dadurch gekennzeichnet, dass bei der Umsetzung von D-Fructose zu Furanderivaten N-Methyl-2-pyrrolidon (NMP) entweder als Reaktions-Lösungsmittel, oder als Co-Lösungsmittel, nämlich als Beimischung zu einem anderen Lösungsmittel, verwendet wird.

In einer besonderen Ausführungsform eines Verfahrens gemäß vorliegender Erfindung wird in Stufe b) NMP als (Co)lösungsmittel, z.B. als Reaktionslösungsmittel oder als Beimischung zu einem anderen Lösungsmittel, verwendet.

In einem Verfahren gemäß vorliegender Erfindung kann, wenn NMP als Lösungsmittel eingesetzt wird, NMP als einziges Lösungsmittel eingesetzt werden, oder NMP wird zusammen mit einem Co-Lösungsmittel verwendet, wobei im Falle der Verwendung eines Co-Lösungsmittels eine NMP-Konzentration von bis zu 70% (v/v), beispielsweise bis zu 60% (v/v) bezogen auf die Gesamtlösungsmittelmenge eingesetzt werden kann. Als Co-Lösungsmittel kommen z.B. Wasser oder organische Lösungsmittel, z.B. solche, die aus dem Stand der Technik bekannt sind, wie N,N-Dimethylsulfoxid (DMSO) oder N,N-Dimethylformamid (DMF) in Betracht.

In einem Verfahren gemäß Stufe B) der vorliegenden Erfindung kann die D-Fructose in einer Menge von bis zu 40% (w/v) eingesetzt werden und wird im allgemeinen in einer Menge von 5 bis 20% eingesetzt, obwohl die Reaktion auch in niedrigerer Konzentration abläuft, beispielsweise in einer D-Fructose Konzentration von (etwa) 1% (w/v). Der Minimalwert ist dabei eher durch die Wirtschaftlichkeit und nicht chemisch definiert.

Saure Katalysatoren in Stufe B) in einem Verfahren nach vorliegender Erfindung schließen übliche saure Katalysatoren, die bei der Umwandlung von Fructose in Furanderivate eingesetzt werden können, ein. Bevorzugt ist der Katalysator eine Brønsted-Säure. Dabei können homogene Säurekatalysatoren, z.B. Schwefelsäure oder Salzsäure, oder heterogene Säurekatalysatoren, beispielsweise Kationenaustauscherharze wie Montmorillonite, bevorzugt Montmorillonit KSF® oder Amberlite, z.B. Amberlite®, bevorzugt Amberlite 15®, verwendet werden. Außerdem können in einem Verfahren nach vorliegender Erfindung Lewis-Säure-Katalysatoren, wie CrCl₂, AlCl₃, SiO₂-MgCl₂ oder ein SILP(*silica supported ionic liquid phase*)-Katalysator eingesetzt werden. Im Allgemeinen liefern diese jedoch nicht so gute Ergebnisse, wie die oben genannten Katalysatoren.

In einem weiteren Aspekt ist ein Verfahren nach vorliegender Erfindung dadurch gekennzeichnet, dass bei der Umsetzung von D-Fructose zu Furanderivaten in Stufe B) als saurer Katalysator
- ein homogener Säurekatalysator, bevorzugt Schwefelsäure oder Salzsäure;
- ein heterogener Säurekatalysator, bevorzugt ein Ionenaustauscher, z.B. ein Montmorillonit, wie Montmorillonit KSF® oder ein Amberlit, wie Amberlite®, bevorzugt Amberlite 15®
- ein Lewis-Säurekatalysator wie z.B. CrCl₂, AlCl₃ oder SiO₂-MgCl₂
- ein SILP-Katalysator,
   bevorzugt ein homogener oder heterogener Säurekatalysator,
eingesetzt wird.

Die Menge eines benötigten Katalysators in Stufe B) kann der Fachmann problemlos durch einfache Vorversuche herausfinden. Die Menge hängt dabei von der Art des verwendeten Katalysators ab.

Beispielhaft sind nachfolgend Katalysator-Mengen bezogen auf die eingesetzte Fructose-Menge angegeben, insbesondere für den Fall, dass NMP als Lösungsmittel verwendet wird:

| Katalysator | Menge |
|---|---|
| IN HCl | 20 bis 200% (v/w) |
| HCl (37%) | 2 bis 25% (v/w) |
| IN H₂SO₄ | 20 bis 200% (v/w) |
| H₂SO₄ conc | 2 bis 25% (v/w) |
| Montmorillonite KSF® | 1 bis 50% (w/w) |
| Amberlite 15® | 1 bis 50% (w/w) |
| CrCl₂, AlCl₃ | 1 bis 20% (w/w) |
| SiO₂-MgCl₂ | 20 bis 200% (w/w) |
| SILP | 10-200% (w/w) |

Dabei sind bei einer Konzentration von etwa 10% (w/v) D-Fructose die angegebenen Werte unproblematisch, bei höheren Fructose-Konzentrationen ist die Menge der Katalysatoren so zu begrenzen, dass die Fructose in der verbleibenden Lösungsmittelmenge noch gelöst werden kann.

Das Verfahren in Stufe B) gemäß vorliegender Erfindung wird bei geeigneten Temperaturen durchgeführt. Geeignete Temperaturen schließen, insbesondere wenn NMP als Lösungsmittel verwendet wird, Temperaturen von 100 bis 220°C, bevorzugt von 115 bis 200°C, insbesondere bevorzugt 135 bis 185°C ein.

Die Reaktionen in Stufe B) unter Verwendung von NMP als Lösungsmittel wurden experimentell durchgehend in geschlossenen Gefäßen (Batch, Mikrowelle) durchgeführt, ohne aktive Druckkontrolle. Aus den Mikrowellenläufen kann man als Maximaldruck bei NMP etwa 2-4 bar ansetzen, stark abhängig von den Additiven. Wird z.B. HCl als Katalysator eingesetzt, so steigt der entstehende Druck auf bis zu 15 bar an. Im kontinuierlichen Betrieb wurde ein konstanter Rückdruck zur Vermeidung des Siedens des Lösemittels, etwa bis zu 40 bar angelegt. Druck entsteht entweder als Dampfdruck von Lösungsmittel(n) oder Additiven oder es wird ein systembedingter (Pump-)Druck angelegt. Für den Reaktionsmechanismus erscheint der Druck aber nicht entscheidend.

Es hat sich herausgestellt, dass das hauptsächlich entstehende Furanderivat in einem Verfahren gemäß vorliegender Erfindung Hydroxymethylfurfural (HMF) der Formel 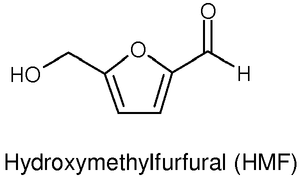 ist.

In einem weiteren Aspekt ist ein Verfahren gemäß vorliegender Erfindung dadurch gekennzeichnet, dass das Furanderivat Hydroxymethylfurfural ist.

In einem Verfahren nach vorliegender Erfindung soll unter "HMF-Selektivität" jener Anteil der verbrauchten D-Fructose verstanden werden, der zu HMF umgesetzt wird.

Furanderivate, die mit einem Verfahren nach vorliegender Erfindung hergestellt werden, können entweder direkt verwendet werden oder in weiteren chemischen Reaktionen zu Folgeprodukten umgesetzt werden. Beispielsweise kann Hydroxymethylfurfural weiter zu 2,5-Furandicarbonsäure (FDCA) der Formel 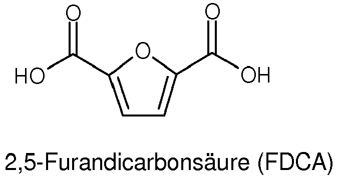 oxidiert werden. FDCA eignet sicht sich bekanntlich als Monomer zur Herstellung von Polymeren wie zum Beispiel Polyethylenfuranoat (PEF), das ähnlich eingesetzt werden kann wie Polyethylenterephthalat (PET), zum Beispiel für Hohlkörper, insbesondere Flaschen wie z.B. Getränke-Flaschen, Flaschen für Kosmetika oder Flaschen für Reinigungsmittel. Bei gleichzeitiger Verwendung von Ethylenglycol aus regenerativen Quellen und FDCA, welches zugänglich ist aus HMF, hergestellt in einem Verfahren gemäß vorliegender Erfindung, kann PEF gewonnen werden, das praktisch vollständig aus nachwachsenden Rohstoffen besteht.

In einem weiteren Aspekt ist die vorliegende Erfindung dadurch gekennzeichnet, dass hergestellte Furanderivate weiter umgesetzt werden, z.B. dass Hydroxymethylfurfural weiter zu 2,5-Furandicarbonsäure oxidiert wird, das gegebenenfalls einer Polymerisation unterworfen wird, z.B. zur Herstellung von Polymeren, wie etwa Polyethylenfuranoat (PEF) eingesetzt wird.

### Beschreibung der Abbildungen

Fig. 1
   Zeigt Ergebnisse bei der Dehydratisierung von D-Fructose in N-Methyl-2-pyrrolidon mit Schwefelsäure als Katalysator gemäß Beispiel 5
Fig. 2 und Fig. 3
   Zeigen Ergebnisse bei der Dehydratisierung von D-Fructose in N-Methyl-2-pyrrolidon mit Schwefelsäure als Katalysator - Durchführung im Mikrowellenreaktor gemäß Beispiel 12
Fig. 4 und Fig. 5
   Zeigen Ergebnisse bei der Dehydratisierung von D-Fructose in N-Methyl-2-pyrrolidon mit Salzsäure als Katalysator - Durchführung im Mikrowellenreaktor gemäß Beispiel 13
Fig. 6
   Zeigt Ergebnisse bei der Dehydratisierung von D-Fructose in N-Methyl-2-pyrrolidon mit Montmorillonit KSF® als Katalysator - Durchführung im Mikrowellenreaktor gemäß Beispiel 14
Fig. 7
   Zeigt Ergebnisse bei der Dehydratisierung von D-Fructose in N-Methyl-2-pyrrolidon mit Salzsäure als Katalysator - Reaktion im Durchflussreaktor gemäß Beispiel 15
Fig. 8
   Zeigt eine Übersicht der getesteten Bedingungen bei der Dehydratisierung von D-Fructose
Fig. 9
   Zeigt einen schematischen Reaktionsaufbau für *stopped flow* Mikrowellenreaktionen und *continuous flow* Reaktionen zur Herstellung von Furanderivaten aus D-Fructose

In den folgenden Beispielen sind alle Temperaturangaben in Grad Celsisus (°C).
Die folgenden Abkürzungen werden verwendet:
- EtOAc: Ethylacetat
- FDCA: Furandicarbonsäure
- h: Stunde(n)
- HMF: 5-Hydroxymethylfurfural
- HPLC: High-performance liquid chromatography
- IPA: Isopropylalkohol (2-Propanol)
- LS: Lävulinsäure
- MeOH: Methanol
- NMP: N-Methylpyrrolidon (N-Methyl-2-pyrrolidon)
- PET: Polyethylenterephthalat
- PEF: Polyethylenfuranoat
- RT: Raumtemperatur
- SILP: Supported Ionic Liquid Phase
- TFA: Trifluoressigsäure

### Beispiel 1

### Umsatz von D-Glucose zu D-Fructose durch eine Xylosereduktase und eine Sorbitoldehydrogenase unter der Verwendung einer Alkoholdehydrogenase zum Recycling des NADPH und einer Lactatdehydrogenase zum Recycling des NAD⁺

Ein 0,5 ml Ansatz enthält 50 mg/ml D-Glucose und 6 U/ml der rekombinanten Xylosereduktase aus *Candida tropicalis* (überexprimiert in *E.coli* BL21 (DE3)) und 0,1 mM NADP⁺. Zur Regeneration des Kofaktors werden 7 % (v/v) IPA und 6 U/ml der rekombinanten Alkoholdehydrogenase aus *Lactobacillus kefir* (überexprimiert in *E.coli* BL21 (DE3)) zugefügt. Die Enzyme werden in Form von Zell-Lysat eingesetzt. Die Reaktion findet für 24 h bei 40° C und pH = 9 (50 mM Tris-HCl-Puffer) unter kontinuierlichem Schütteln (900 rpm) in einem offenen System statt. Das offene System führt zur Entfernung des gebildeten Acetons, was die Reaktion in Richtung D-Sorbitol-Bildung treibt. Im offenen System verdampfen Wasser und IPA ebenfalls, sodass diese nach 6 h und 21 h nachdosiert werden. Dabei werden jeweils wieder ein Gesamtvolumen von 0,5 ml sowie eine IPA-Konzentration von 7 % (v/v) eingestellt. Nach 24 h wird das Reaktionsgefäß bei 60°C unter Vakuum inkubiert, um die Enzyme zu deaktivieren und die organischen Lösungsmittel zu verdampfen. Nach dem Abkühlen auf RT werden die rekombinante D-Sorbitoldehydrogenase aus *Bacillus subtilis* (überexprimiert in *E.coli* BL21 (DE3)) in einer Endkonzentration von 5 U/ml, ZnCl₂ in einer Endkonzentration von 1 mM und NAD⁺ in einer Endkonzentration von 0,1 mM zugefügt. Zur Kofaktor-Regenerierung werden 5 U/ml (Endkonzentration) Laktat-Dehydrogenase aus Kaninchenmuskel (Sigma Aldrich) und 300 mM Pyruvat eingesetzt. Der Ansatz wird auf 0,5 ml mit Wasser aufgefüllt. Die Reaktion findet für weitere 24 h bei 40°C unter kontinuierlichem Schütteln (900 rpm) im geschlossenen System statt. Es wird ein Umsatz der D-Glucose zu D-Fructose von >90% erreicht.

### Beispiel 2

### Umsatz der D-Glucose zu D-Fructose durch eine Xylosereduktase und eine Sorbitoldehydrogenase unter der Verwendung einer Alkoholdehydrogenase zum Recycling des NADPH und einer Oxidase zum Recycling des NAD⁺

Ein 0,5 ml Ansatz enthält 50 mg/ml D-Glucose, 6 U/ml der rekombinanten Xylosereduktase aus *Candida tropicalis* (überexprimiert in *E.coli* BL21 (DE3)) und 0,1 mM NADP⁺. Zur Regeneration des Kofaktors werden 7 % (v/v) IPA und 6 U/ml der rekombinanten Alkoholdehydrogenase aus *Lactobacillus kefir* (überexprimiert in *E.coli* BL21 (DE3)) zugefügt. Die Enzyme werden in Form von Zell-Lysat eingesetzt. Die Reaktion findet für 24 h bei 40°C und pH = 8 (50 mM Tris-HCl-Puffer) unter kontinuierlichem Schütteln (900 rpm) in einem offenen System statt. Das offene System führt zur Entfernung des entstehenden Acetons, was die Reaktion Richtung D-Sorbitol-Bildung treibt. Im offenen System verdampfen Wasser und IPA ebenfalls, sodass diese nach 6 h und 21 h nachdosiert werden. Dabei werden jeweils wieder ein Gesamtvolumen von 0,5 ml sowie eine IPA-Konzentration von 7 % (v/v) eingestellt. Nach 24 h wird das Reaktionsgefäß bei 60°C unter Vakuum inkubiert um die Enzyme zu deaktivieren und IPA, sowie entstandenes Aceton zu verdampfen. Nach dem Abkühlen auf Raumtemperatur werden die rekombinante D-Sorbitoldehydrogenase aus *Bacillus subtilis* (überexprimiert in *E.coli* BL21 (DE3)) in einer Endkonzentration von 5 U/ml, CaCl₂ in einer Endkonzentration von 1 mM und eine Mischung (1:1) aus NAD⁺ und NADH in einer Endkonzentration von 0,1 mM zugefügt. Zur Kofaktor-Regenerierung werden 10 U/ml (Endkonzentration) der NADH-Oxidase aus *Leuconostoc mesenteroides* (überexprimiert in *E.coli* BL21 (DE3)) eingesetzt. Die Enzyme werden in Form von Zell-Lysat eingesetzt. Der Ansatz wird auf 0,5 ml mit Wasser aufgefüllt. Die Reaktion findet für weitere 24 h bei 40°C unter kontinuierlichem Schütteln (900 rpm) im offenen System statt, um genug Sauerstoffversorgung für die NADH-Oxidase aus der Luft zu gewährleisten. Im offenen System bei 40°C verdampft Wasser. Es wird daher nach 6 h und 21 h mit Wasser auf 0,5 ml aufgefüllt. Es wird ein Umsatz der D-Glucose zu D-Fructose von ca. 98% erreicht.

### Beispiel 3

### Aufarbeitung und Analytik der Zucker

Der Ansatz wird für 10 min bei 65°C inkubiert um die Enzyme zu deaktivieren und anschließend zentrifugiert. Der Überstand wird dann über einen 0,2 µM PVDF Filter filtriert und mittels Ligand-Exchange-HPLC analysiert (Agilent Technologies, Inc.). Aufgetrennt werden Zucker und Polyole dabei über eine Blei-Säule von Showa Denko K.K. (Shodex® Sugar SP0810) mit einem Fluss von 0,5 ml/min Wasser (VWR International GmbH, HPLC Grade) bei 80°C. Die Detektion erfolgt mittels Lichtbrechungsdetektor (RID, Agilent 1260 Infinity®, Agilent Technologies, Inc.). Es wird ein Inlinefilter von Agilent Technologies, Inc. sowie als Vorsäulen eine Anionen-Austauscher-Säule (Shodex® Axpak-WAG), eine Reversed-Phase Säule (Shodex® Asahipak® ODP-50 6E) und eine Zucker-Vorsäule (SUGAR SP-G), jeweils von Showa Denko K.K., verwendet.

### Beispiel 4

### Materialien und Methoden für die Umsetzung von D-Fructose zu Furanderivaten

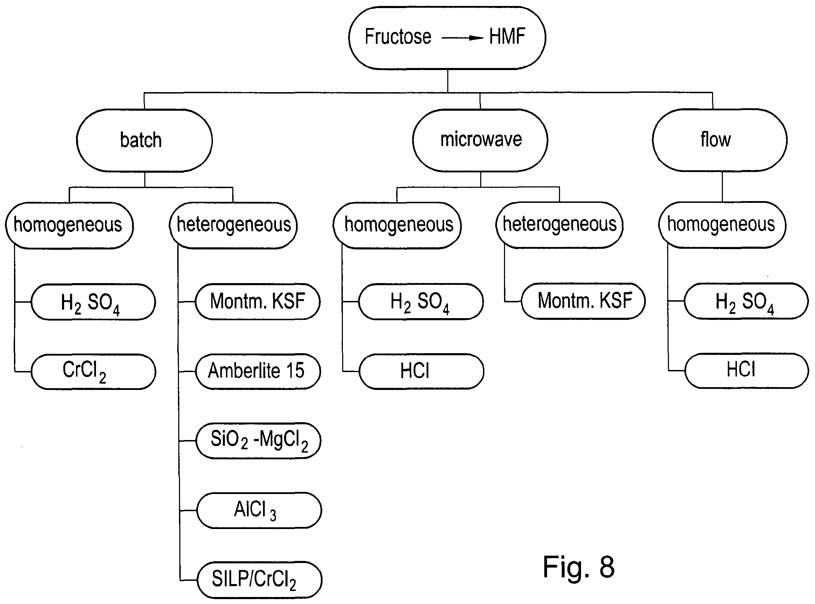

Im Rahmen dieser Erfindung wurden Dehydratationsreaktionen von D-Fructose zu HMF unter verschiedenen Reaktionsbedingungen durchgeführt, wahlweise als Standart-Batch-Prozess, unter Mikrowellen-assistierter Erhitzung oder mittels "continuous flow"-Bedingungen. Fig. 8 zeigt eine Übersicht über die getesteten Bedingungen. Überraschenderweise wurde gefunden, dass NMP als Lösungsmittel bei der Reaktion im Vergleich zu bisher bekannten Systemen in Kombination mit homogenen oder heterogenen Katalysatoren sowohl im Mikrowellen-assistierten-Verfahren als auch unter "continuous flow"-Bedingungen höhere Umsätze liefert.

### Synthese von SiO₂-MgCl₂

SiO₂-MgCl₂ wurde hergestellt in Anlehnung an eine Vorschrift von Yasuda et al. (Yasuda, M.; Nakamura, Y.; Matsumoto, J.; Yokoi, H. Shiragami, T. Bull. Chem. Soc. Jpn. 2011, 84, 416-418).

### Synthese von SILPs

Der SILP Katalysator wurde entsprechend bekannter Vorschriften (Fu, S.-K.; Liu, S.-T. Synth. Commun. 2006, 36, 2059-2067) unter Verwendung von N-Methylimidazol hergestellt. Zur Immobilisierung wurde die erhaltene ionische Flüssigkeit mit 200 Gew.-% Silica-Gel in trockenem Chloroform (100 mL pro 10 g SiO₂) vermischt und 24 h auf 70 °C erhitzt. Der erhaltene Feststoff wurde abfiltriert, mit Chloroform gewaschen und unter reduziertem Druck getrocknet. Das erhaltene Silica-Gel wies eine Beladung von ca. 16 Gew.-% an Katalysator auf.

### Allgemeine Bedingungen Batch Reaktionen

Falls nicht anders beschrieben wurden alle Batch-Reaktionen im 4 mL Schraubdeckelglas durchgeführt. Die Erwärmung wurde in geeigneten Aluminiumblöcken bis zur gewünschten Temperatur durchgeführt.

### Mikrowellenreaktionen im Batch-Verfahren

Mikrowellen-Reaktionen wurden im Batch-Verfahren an einer Biotage-Initiator Sixty laboratory Mikrowelle durchgeführt, die mit einem Autosampler ausgerüstet war, um sequentielle Reaktionsführungen zu ermöglichen. Das Absorbtionslevel wurde auf Maximalwert eingestellt, wodurch die maximale Energiezufuhr automatisch auf 400 W geregelt wurde.

### Stopped flow Mikrowellenreaktionen und continuous-flow Reaktionen

Stopped flow Reaktionen zur Optimierung einer semi-kontinuierlichen Prozessführung wurden an einem an einem CEM® Discover System mit CEM® Voyager Upgrade und mittels externem Drucksensor durchgeführt. Für Reaktionen in kontinuierlicher Prozessführung wurde ein kartuschenbasiertes Reaktor System X-Cube von ThalesNano®, ausgerüstet mit einem Gilson® GX-271 Autosampler zur automatischen Produktsammlung, verwendet. Zwei Quarzsand Kartuschen (CatCart®, 70 x 4 mm) wurden hierbei als Reaktionszone eingebaut.

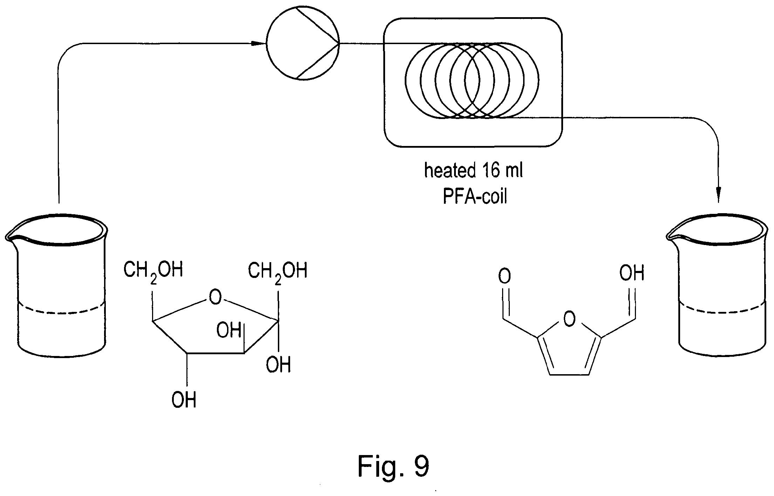

Alternativ wurde eine Perfluoralkoxyalkan-Kapillare eingesetzt (PFA-Kapillare, 0.8 mm Innendurchmesser, 1.6 mm Außendurchmesser), welche um einen beheizbaren Aluminiumzylinder gewickelt wurde. Die Substrate wurden mittels einer Shimadzu LC-10AD HPLC Pumpe in der gewünschten Flussrate zugegeben. Exakte Volumina (Säule 16,0 mL; Totvolumen vor und nach der Säule jeweils 1,0 mL) wurden bestimmt, in dem definierte Flussraten des reinen Lösungsmittels mit einer Digitalstoppuhr verfolgt wurden. Der Reaktionsaufbau ist in Fig. 9 dargestellt.

### Analyse der Reaktionen zur Umsetzung von D-Fructose zu Furanderivaten

Zur quantitativen HPLC-Analyse wurden Proben der Reaktionssamples (22 µL falls nicht anders angegeben) mit entionisiertem Wasser auf 1 mL verdünnt. Bei Reaktionsproben, die eine unterschiedliche Konzentration aufwiesen, wurde die Verdünnung so angepasst, dass die maximale Konzentration 2 mg/ml nicht überschritt.

Zu dieser Lösung wurden 100 µL 3-Hydroxybenzylalkohol als interner Standard hinzugegeben, woraufhin die Probe gründlich durchmischt wurde. Feste Rückstände wurden mittels Zentrifugieren (5 min, 20000 G) oder Filtration (Phenex PTFE, 4 mm, 0.2 µm) abgetrennt. Quantifizierung erfolgte ausgehend von den Flächen der Peaks im RI-Spektrum im Vergleich zum internen Standard.

Die Proben wurden mittels HPLC an einem Thermo Scientific® Surveyor Plus System oder einem Shimadzu® Nexera System jeweils ausgestattet mit PDA Plus- und RI Detektor analysiert. Für die Trennung wurde als stationäre Phase eine Ionenauschlusssäule von Phenomenex® (Rezex RHM-Monosaccharide H+ (8%), 150 x 7.8 mm, aufgebaut aus quervernetzter Matrix von sulfoniertem Styrol und Divinylbenzol, H⁺-Form) und ein Laufmittelgemisch aus Wasser (HPLC-grade) und 0,1 % TFA (HPLC-grade) als Eluent verwendet. Die Säulentemperatur wurde konstant und auf 85 °C gehalten, wobei die Laufzeit auf 25 Minuten optimiert wurde. Produktquantifizierung wurde anhand eines internen Standards durch Integration des RI-Signals durchgeführt. Mittels PDA wurden zusätzlich die Wellenlängen 200 nm, 254 nm und 280 nm zur weiteren Reaktionsanalyse aufgezeichnet.

### GP1 - D-Fructose-Dehydratisierung im Batch-Verfahren

In einer Standardreaktion zur Reaktionsoptimierung wurden 100 mg D-Fructose (0,56 mmol) und der jeweilige Katalysator in der gewünschten Menge in ein Glasvial gegeben und mit 1 mL frisch destilliertem NMP versetzt. Die erhaltenen Lösung/Suspension wurde auf die gewählte Temperatur erhitzt und für die gewünschte Zeit reagieren gelassen.

### GP2 - D-Fructose-Dehydratisierung im Mikrowellen-Batch-Verfahren

In einer Standardreaktion zur Reaktionsoptimierung wurden 100 mg D-Fructose (0,56 mmol) und der jeweilige Katalysator in der gewünschten Menge in ein Mikrowellen-Gefäß (0,5 - 2,0 mL) gegeben. Das Gefäß wurde mit einem Rührmagneten ausgestattet und mit 1 mL NMP aufgefüllt. Die Strahlungsintensität der Mikrowelle wurde von einem firmeneigenen Regulationsalgorithmus automatisch eingestellt, um die gewünschte Temperatur zu erreichen. Eine schnelle Kühlung des Reaktionsgefäßes wurde mittels eingeblasener Druckluft von mindestens 6 bar realisiert.

### GP3 - D-Fructose-Dehydratisierung im Mikrowellen stoppedflow Verfahren

In einer Standardreaktion zur Reaktionsoptimierung wurden eine D-Fructose Standardlösung (1 mL; c = 100 mg/mL in NMP) und Salzsäure (100 µL; c = 1 mol/L) in ein Mikrowellengefäß gefüllt und mit einem Rührmagneten versehen. Nach Versiegelung des Vials mittels Snap-Cap wurde die Lösung für die gewünschte Zeit auf die gewünschte Temperatur erhitzt. Um eine schnellstmögliche Erwärmung herbeizuführen wurde die zugeführte Energie entsprechend der nachfolgenden Tabelle 1 eingestellt.

**Tabelle 1**

| Leistungseinstellung der Mikrowelle und zugehörige Temperaturen | | | |
|---|---|---|---|
| **Temperatur** | **Leistungseinstellung** | **Temperatur** | **Leistungseinstellung** |
| 100°C | 50 W | 180°C | 125 W |
| 125°C | 65 W | 200°C | 140 W |
| 150°C | 100 W | 220°C | 160 W |

Eine schnelle Kühlung des Reaktionsgefäßes wurde mittels eingeblasener Druckluft von mindestens 6 bar realisiert.

### GP4 - D-Fructose-Dehydratisierung im Kartuschen-basierten Reaktorsystem

In einer Standardreaktion zur Reaktionsoptimierung wurde eine D-Fructose Standardlösung (1 mL; c = 100 mg/mL in NMP) mit Salzsäure (c = 1 mol/L) gemischt und durch eine Reagenzpumpe in das Reaktionssystem gepumpt. Während des Aufheizprozesses wurden mehrere Vorproben entnommen, um eine stabile Temperatur und eine stabile Flussrate zu überwachen. Als Reaktionstemperatur wurden 150°C, 180°C und 200°C gewählt, wobei der Reaktionsdruck auf 40 bar reguliert wurde. Hierfür wurden Flussraten zwischen 0,2 und 0,6 ml/min gewählt. Reaktionsproben wurden in 2,5 mL Mengen aufgefangen und analysiert.

### Beispiel 5

### Verwendung von Schwefelsäure als Katalysator zur Dehydratisierung von D-Fructose

Es wurden verschiedene Temperaturen, Reaktionszeiten und Säurekonzentrationen verglichen. Die Reaktionen wurden nach "GP1" (Beispiel 4) durchgeführt. Als Katalysator wurde entweder 100 µl 1 N Schwefelsäure oder 10 µl konzentrierte Schwefelsäure verwendet. In Tabelle 2 sind die Ergebnisse zusammengefasst.

**Tabelle 2**

| Schwefelsäure als Katalysator zur Dehydratisierung von D-Fructose | | | | | | |
|---|---|---|---|---|---|---|
| **Katalysator** | **Temperatur** | **Reaktionszeit** | **Fructose-Verbrauch** | **HMF Ausbeute** | **HMF Selektivität** | **LS Ausbeute** |
| IN H₂SO₄ | 100°C | 3 h | 69% | 45% | 65% | < 1% |
| 1N H₂SO₄ | 120°C | 4 h | 95% | 77% | 81% | < 1% |
| 1N H₂SO₄ | 150°C | 15 min | 98% | 88% | 90% | < 1% |
| 1N H₂SO₄ | 180°C | 10 min | 100% | 85% | 85% | < 1% |
| H₂SO₄ conc. | 120°C | 45 min | 98% | 85% | 90% | < 1% |
| H₂SO₄ conc. | 150°C | 10 min | 100% | 90% | 90% | < 1% |
| H₂SO₄ conc. | 180°C | 5 min | 100% | 82% | 82% | < 1% |

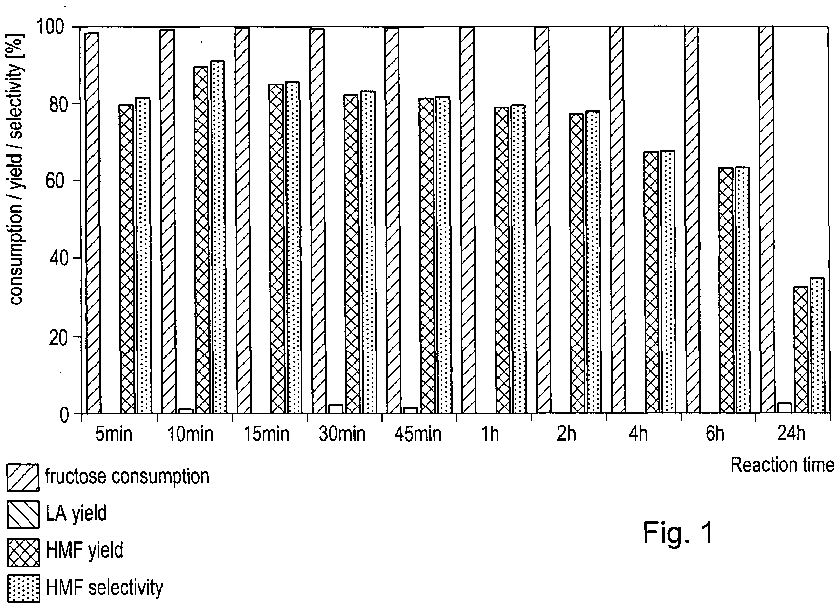

Die Bildung schwarzer unlöslicher Polymere und Humine wurde unter den verwendeten optimalen Bedingungen nicht beobachtet. Um den Verlauf der Reaktion zu analysieren wurde eine Zeitreihe für eine repräsentative Reaktion aufgenommen (H₂SO₄ conc., 150°C, siehe Fig. 1.

### Beispiel 6

### Verwendung von Chrom-(II)-chlorid als Katalysator zur Dehydratisierung von D-Fructose

Wie von Zhao, H.; Holladay, J. E.; Brown, H.; Zhang, Z. C. Science 2007, 316, 1597-1600 beschrieben, kann Chrom-(II)-chlorid als effizienter Katalysator zur Dehydratisierung von D-Fructose eingesetzt werden. In diesem Beispiel ist der Effekt CrCl₂ in N-Methyl-2-pyrrolidon gezeigt. Die Experimente wurden nach Vorschrift "GP1" (Beispiel 4) durchgeführt. Während relative geringe Ausbeuten an HMF erreicht wurden, konnten signifikante Mengen an teerartigen Verbindungen beobachtet werden (Tabelle 3).

**Tabelle 3**

| Chrom-(II)-chlorid als Katalysator zur Dehydratisierung von D-Fructose | | | | | | |
|---|---|---|---|---|---|---|
| **Menge Katalysator** | **Temp.** | **Reaktionszeit** | **Fructose-Verbrauch** | **HMF Ausbeute** | **HMF Selektivität** | **LS Ausbeute** |
| 10 mg CrCl₂ | 100°C | 3 h | 86% | 51% | 59% | < 1% |
| 10 mg CrCl₂ | 150°C | 3h | 100% | 39% | 39% | < 1% |

### Beispiel 7

### Verwendung von Montmorillonit KSF® als Katalysator zur Dehydratisierung von D-Fructose

100 mg D-Fructose wurde in Gegenwart von 1 ml N-Methyl-2-pyrrolidon unter Rühren inkubiert (Vorschrift "GP1", Beispiel 4). Als Reaktionszeit wurde einheitlich 3 h gewählt. Dabei wurden unterschiedliche Mengen an Montmorrilonite KSF® als Katalysator zugegeben. Tabelle 4 fasst die Ergebnisse zusammen. Unter den besten Bedingungen konnte eine HMF-Ausbeute von 61 % bei einer HMF-Selektivität von 63 % erreicht werden.

**Tabelle 4**

| Montmorillonit KSF® als Katalysator zur Dehydratisierung von D-Fructose | | | | | | |
|---|---|---|---|---|---|---|
| **Katalysator** | **Temp.** | **Fructose-Verbrauch** | **HMF Ausbeute** | **HMF Selektivität** | **LS Ausbeute** | **Teer** |
| 1 mg | 120°C | 37% | 11% | 31% | < 1% | nein |
| 3 mg | 120°C | 54% | 20% | 38% | < 1% | nein |
| 5 mg | 120°C | 65% | 30% | 46% | < 1% | nein |
| 7 mg | 120°C | 73% | 32% | 44% | < 1% | nein |
| 10 mg | 120°C | 80% | 41% | 52% | < 1% | nein |
| 20 mg | 120°C | 90% | 43% | 48% | < 1% | nein |
| 40 mg | 120°C | 94% | 43% | 46% | < 1% | nein |
| 1 mg | 130°C | 31% | 11% | 35% | < 1% | nein |
| 3 mg | 130°C | 73% | 35% | 48% | < 1% | nein |
| 5 mg | 130°C | 87% | 46% | 53% | < 1% | nein |
| 7 mg | 130°C | 92% | 50% | 55% | < 1% | nein |
| 10 mg | 130°C | 94% | 49% | 52% | < 1% | nein |
| 20 mg | 130°C | 96% | 54% | 57% | < 1% | nein |
| 40 mg | 130°C | 97% | 54% | 55% | < 1% | ja |
| 1 mg | 140°C | 72% | 30% | 42% | < 1% | nein |
| 3 mg | 140°C | 91% | 46% | 51% | < 1% | nein |
| 5 mg | 140°C | 95% | 53% | 56% | < 1% | nein |
| 7 mg | 140°C | 96% | 53% | 55% | < 1% | nein |
| 10 mg | 140°C | 98% | 55% | 56% | < 1% | nein |
| 20 mg | 140°C | 98% | 56% | 57% | < 1% | nein |
| 40 mg | 140°C | 99% | 56% | 56% | < 1% | ja |
| 1 mg | 150°C | 94% | 44% | 46% | < 1% | nein |
| 3 mg | 150°C | 96% | 52% | 54% | < 1% | nein |
| 5 mg | 150°C | 98% | 56% | 57% | < 1% | nein |
| 7 mg | 150°C | 98% | 57% | 59% | < 1% | nein |
| 10 mg | 150°C | 98% | 58% | 59% | < 1% | ja |
| 20 mg | 150°C | 97% | 61% | 63% | < 1% | ja |
| 40 mg | 150°C | 97% | 61% | 63% | < 1% | ja |

### Beispiel 8

### Verwendung von Amberlite 15® als Katalysator zur Dehydratisierung von D-Fructose

Dieses Beispiel zeigt die Verwendung eines starken Ionenaustauschers mit Sulfonsäureresten basieren auf einem makrovernetzen Harz. 100 mg D-Fructose wurde in Gegenwart von 1 ml N-Methyl-2-pyrrolidon 3 h bei 100°C unter Rühren inkubiert (Vorschrift "GP1, Beispiel 4). Dabei wurde Amberlite 15® als Katalysator zugegeben. In Tabelle 5 ist das Ergebnis dieses Experiments gezeigt. Im Gegensatz zu Montmorillonit KSF® konnte bei der relativ niedrigen Temperatur eine höhere Ausbeute erreicht werden. Die Bildung von teerartigen Verbindungen wurde vermieden.

**Tabelle 5**

| Amberlite 15® als Katalysator zur Dehydratisierung von D-Fructose | | | | | | |
|---|---|---|---|---|---|---|
| **Menge Katalysator** | **Temp.** | **Reaktionszeit** | **Fructose-Verbrauch** | **HMF Ausbeute** | **HMF Selektivität** | **LS Ausbeute** |
| 10 mg | 100°C | 3h | 70% | 50% | 71% | < 1% |

### Beispiel 9

### Verwendung von SiO₂-MgCl₂ als Katalysator zur Dehydratisierung von D-Fructose

Da ein Silicagel-Magnesiumchlorid-Komplex katalytische Aktivität bei der Dehydratisierung von Kohlenhydraten in Acetonitril zeigte (Yasuda, M.; Nakamura, Y.; Matsumoto, J.; Yokoi, H. Shiragami, T. Bull. Chem. Soc. Jpn. 2011, 84, 416-418), wurde dieser Katalysator auf seine Eignung in N-Methyl-2-pyrrolidon getestet. Unter Reaktionsbedingungen wie in "GP1" (Beispiel 4) wurde im besten Fall eine Ausbeute von 26% HMF erreicht (siehe Tabelle 6). Wurde allerdings lediglich Silica-Gel verwendet, sank die Ausbeute auf unter 1 %. Dabei wurde die Bildung großer Mengen teerartiger Verbindungen beobachtet.

**Tabelle 6**

| SiO₂-MgCl₂ als Katalysator zur Dehydratisierung von D-Fructose | | | | | | |
|---|---|---|---|---|---|---|
| **Menge Katalysator** | **Temp.** | **Reaktionszeit** | **Fructose-Verbrauch** | **HMF Ausbeute** | **HMF Selektivität** | **LS Ausbeute** |
| 200 mg | 150°C | 30 min | 99% | 26% | 26% | 4% |

### Beispiel 10

### Verwendung von AlCl₃ als Katalysator zur Dehydratisierung von D-Fructose

AlCl₃ wurde als Beispiel für einen Lewissäure-Katalysator unter Reaktionsbedingungen "GP1" (Beispiel 4) getestet. Dazu wurde frisch sublimiertes **AlCl₃** verwendet. Es wurden ähnliche Ergebnisse wie mit Amberlite 15® erzielt. Der Katalysator ist allerdings Hydrolyse-empfindlich und kann daher nicht für wiederholte Anwendungen oder in kontinuierlichen Prozessen eingesetzt werden. Es entstanden außerdem größere Mengen an teerartigen Verbindungen (Ergebnis siehe Tabelle 7).

**Tabelle 7**

| AlCl₃ als Katalysator zur Dehydratisierung von D-Fructose | | | | | | |
|---|---|---|---|---|---|---|
| **Menge Katalysator** | **Temp.** | **Reaktionszeit** | **Fructose-Verbrauch** | **HMF Ausbeute** | **HMF Selektivität** | **LS Ausbeute** |
| 10 mg | 100°C | 3h | 100% | 50% | 50% | < 1% |

### Beispiel 11

### Verwendung von SILPs kombiniert mit Chrom-(II)-chlorid als Katalysator zur Dehydratisierung von D-Fructose

Unter Anwendung der Reaktionsbedingungen "GP1" (Beispiel 4) wurde eine Kombination aus CrCl₂ und SILPs *(silica-supported ionic liquid phase,* siehe Beispiel 4) getestet. Nach 20 min konnte eine Ausbeute von fast 50 % HMF erreicht werden. Diese konnte allerdings bei längeren Reaktionszeiten nicht gesteigert werden. Außerdem konnte bei kürzeren Reaktionszeiten eine Umwandlung von D-Fructose zu D-Glucose festgestellt werden (Tabelle 8).

**Tabelle 8**

| SILPs kombiniert mit CrCl₂ als Katalysator zur Dehydratisierung von D-Fructose | | | | | | |
|---|---|---|---|---|---|---|
| **Temp.** | **Reaktionszeit** | **Fructose-Verbrauch** | **Glucose-Ausbeute** | **HMF Ausbeute** | **HMF Selektivität** | **LS Ausbeute** |
| 120°C | 5 min | 85% | 5% | 39% | 46% | < 1% |
| 120°C | 10 min | 94% | 3% | 45% | 48% | < 1% |
| 120°C | 15 min | 99% | 1% | 44% | 45% | < 1% |
| 120°C | 20 min | 97% | 2% | 49% | 51% | < 1% |
| 120°C | 25 min | 97% | 1% | 47% | 48% | < 1% |
| 120°C | 30 min | 98% | < 1% | 49% | 50% | < 1% |
| 120°C | 45 min | 99% | < 1% | 48% | 49% | < 1% |
| 120°C | 1 h | 99% | < 1% | 52% | 52% | < 1% |

### Beispiel 12

### Verwendung von Schwefelsäure als Katalysator zur Dehydratisierung von D-Fructose (Mikrowellen-Erhitzung)

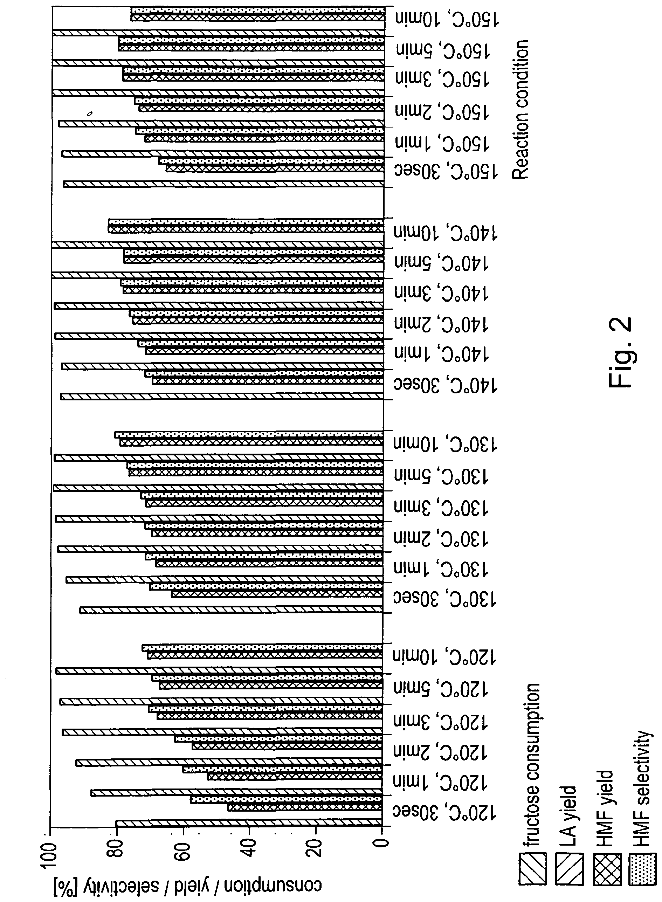

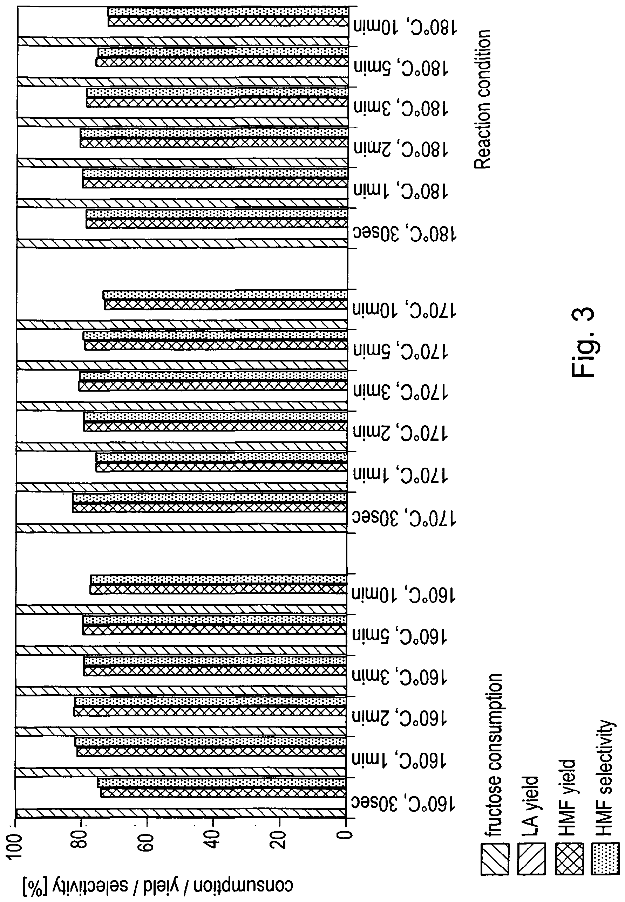

Um eine bessere Kontrolle über Aufheizphase und Abkühlphase sowie über die Reaktionstemperatur zu erreichen, wurde ein Mikrowellen-basiertes System zur TemperaturEinstellung angewendet. Unter Verwendung von N-Methyl-2-pyrrolidon wurden Proben wie in Vorschrift "GP2" (Beispiel 4) beschrieben hergestellt. Unter den verwendeten Reaktionsbedingungen wurde keine Bildung von teerartigen Verbindungen festgestellt. Es konnte maximal ein vollständiger Umsatz von D-Fructose und eine Ausbeute von 83 % HMF erreicht werden (Fig. 2 und Fig. 3).

### Beispiel 13

### Verwendung von Salzsäure als Katalysator zur Dehydratisierung von D-Fructose (Mikrowellen-Erhitzung)

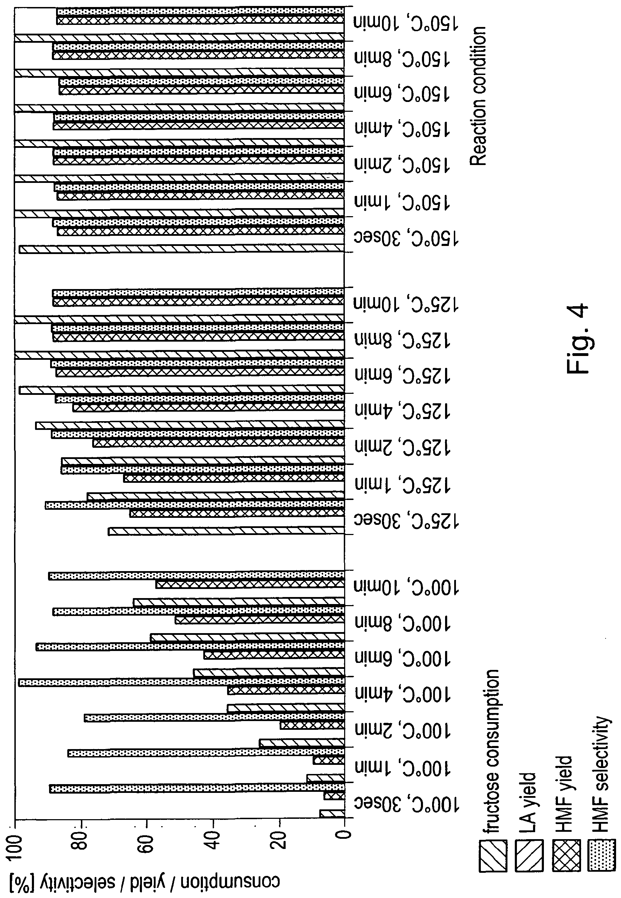

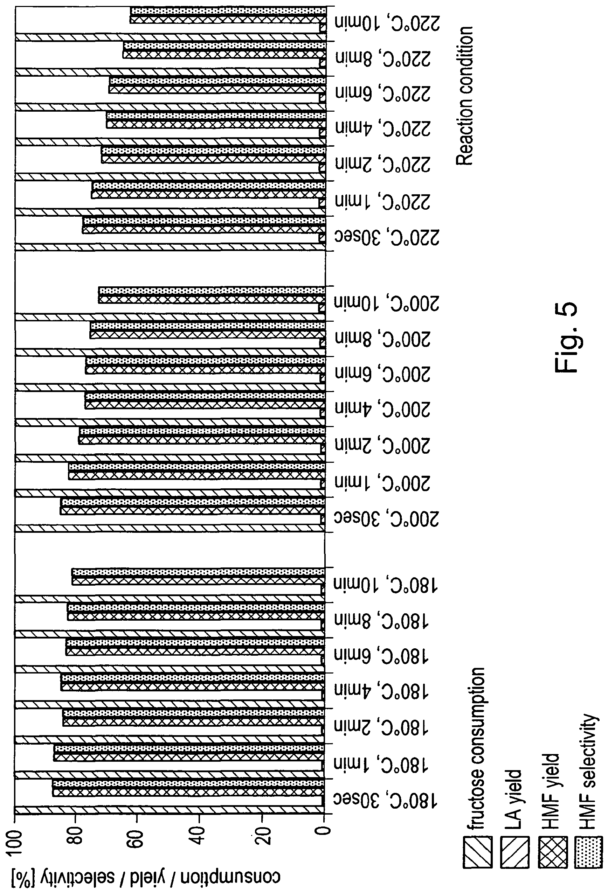

Die Dehydratisierung von D-Fructose wurde in einem *stopped-flow* Mikrowellenreaktor nach Vorschrift "GP3" (Beispiel 4) durchgeführt. Höhere Temperaturen waren notwendig, um einen vollständigen Umsatz von D-Fructose zu erreichen. Während bei niedrigeren Temperaturen längere Reaktionszeiten die Ausbeute von HMF verbesserten, sank diese bei höheren Temperaturen mit steigender Reaktionszeit (Abbildungen 4 und 5. Bei vollständigem Umsatz von D-Fructose konnte eine maximale Ausbeute von 89 % HMF erreicht werden.

### Beispiel 14

### Verwendung von Montmorillonit KSF® als Katalysator zur Dehydratisierung von D-Fructose (Mikrowellen-Erhitzung)

Da mit Mikrowellen-Verfahren eine schnelle Aufheizung/Abkühlung sowie eine sehr gute Kontrolle der Temperatur im Reaktionsgefäß erfolgen kann, wurde auch der heterogene Katalysator Montmorillonit KSF® zur Dehydratisierung von D-Fructose in N-Methyl-2-pyrrolidon verwendet. Es wurden Reaktionsbedingungen nach "GP2" (Beispiel 4) verwendet. Die Reaktionszeit lag bei 5 min. Obwohl lediglich vergleichsweise geringe D-Fructose-Umsätze und HMF-Ausbeuten erreicht wurden, konnte die Bildung von teerartigen Verbindungen vermieden werden (Ergebnisse siehe Tabelle 9).

**Tabelle 9**

| Montmorillonit KSF® als Katalysator zur Dehydratisierung von D-Fructose (Mikrowellen-Erhitzung) | | | | | | |
|---|---|---|---|---|---|---|
| **Menge Katalysator** | **Temp.** | **Fructose Verbrauch** | **HMF Ausbeute** | **HMF Selektivität** | **LS Ausbeute** | **Teer** |
| 5 mg | 150°C | 51% | 20% | 39% | < 1% | nein |
| 7 mg | 150°C | 61% | 26% | 43% | < 1% | nein |
| 10 mg | 150°C | 64% | 30% | 46% | < 1% | nein |
| 15 mg | 150°C | 76% | 38% | 50% | < 1% | nein |
| 20 mg | 150°C | 82% | 43% | 52% | < 1% | nein |

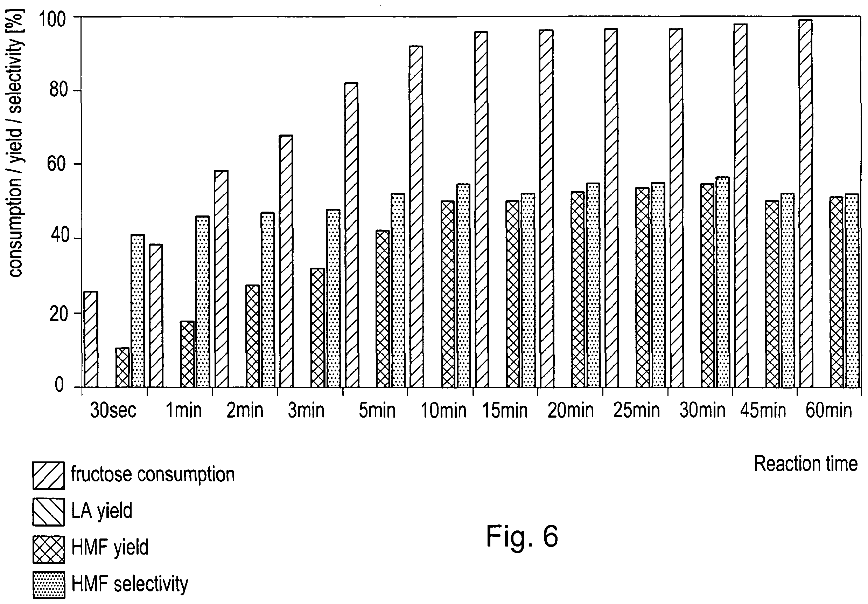

Um die besten Reaktionsbedingungen zu finden wurden verschiedene Reaktionszeiten unter Verwendung von 20 mg Katalysator bei 150°C getestet (Abbildung 6).

### Beispiel 15

### Verwendung von Schwefelsäure zur Katalyse der Umsetzung von D-Fructose zu Furanderivaten (kontinuierlicher Prozess)

D-Fructose (10 % w/v) und konzentrierte Schwefelsäure (1 % v/v) wurden in N-Methyl-2-pyrrolidon gelöst. Die Mischung wurde mittels einer PFA-Kapillare unter kontinuierlichem Fluss durch den Reaktor gepumpt (Reaktionstemperatur 150°C). Nachdem die ersten 18 ml verworfen wurden, wurden weitere 10 ml zur Analyse gesammelt. Durch eine Reihe von Flussraten wurde der Effekt unterschiedlicher Verweilzeiten im Reaktor getestet (Tabelle 10).

**Tabelle 10**

| Schwefelsäure zur Katalyse der Umsetzung von D-Fructose zu Furanderivaten (kontinuierlicher Prozess) | | | | | |
|---|---|---|---|---|---|
| **Flussrate (ml/min)** | **Verweilzeit** | **Fructose Verbrauch** | **HMF Ausbeute** | **HMF Selektivität** | **LS Ausbeute** |
| 0.8 ml/min | 20 min | 100% | 74% | 74% | < 1% |
| 1.6 ml/min | 10 min | 100% | 75% | 75% | < 1% |
| 3.2 ml/min | 5 min | 100% | 76% | 76% | < 1% |

Unter den untersuchten Bedingungen wurde keine Bildung von schwarzen unlöslichen Polymeren und Huminen beobachtet.

### Beispiel 16

### Verwendung von Salzsäure zur Katalyse der Umsetzung von D-Fructose zu Furanderivaten (kontinuierlicher Prozess)

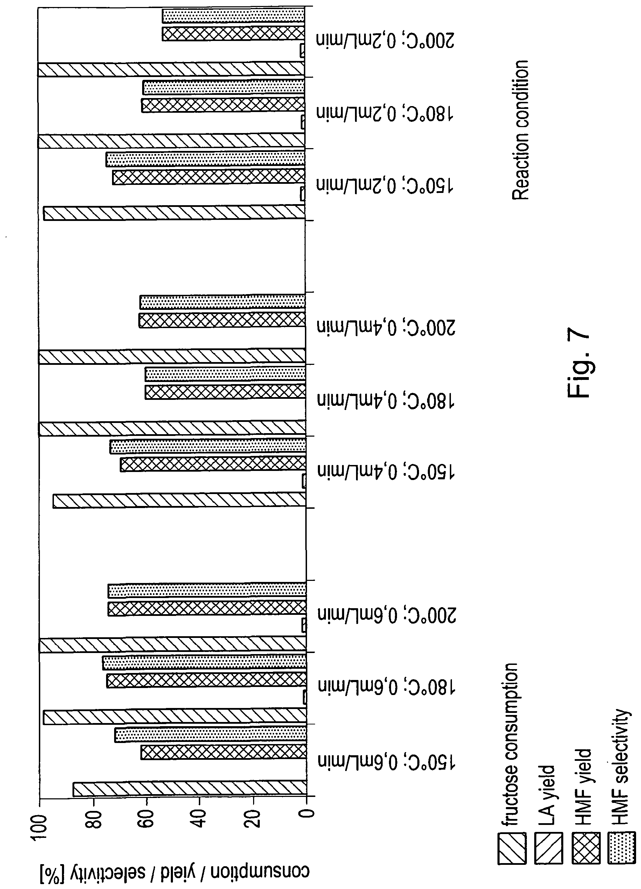

In diesem Beispiel wurde Salzsäure als Katalysator zur Dehydratisierung von D-Fructose in NMP unter kontinuierlichem Fluss eingesetzt (Reaktionsbedingungen siehe Vorschrift "GP4", Beispiel 4). Eine maximale Ausbeute von 75 % HMF konnte bei einer Reaktionstemperatur von 180°C und einem Fluss von 0,6 ml/min erreicht werden. Dabei wurde eine Selektivität von 76 % HMF erreicht. Meist lag der Anteil von Lävulinsäure (LS) unter 1 % (Ergebnisse siehe Fig. 7).

## Patentansprüche

1. Verfahren zur Gewinnung von Furanderivaten aus D-Glucose, **dadurch gekennzeichnet, dass**
A) D-Glucose in einem enzymatischen Verfahren unter Verwendung und Regenerierung von Redoxkofaktoren in D-Fructose übergeführt wird,
wobei D-Glucose unter Beteiligung von zwei oder mehr Oxidoreduktasen in Produktbildungsreaktionen in D-Fructose überführt wird und bei der Umwandlung von D-Glucose zu D-Fructose zuerst eine enzymatisch katalysierte Reduktion zu D-Sorbitol und anschließend eine enzymatisch katalysierte Oxidation von D-Sorbitol zu D-Fructose durchgeführt werden,
wobei als Redoxkofaktoren NAD⁺/NADH und NADP⁺/NADPH verwendet werden und als Resultat mindestens zweier weiterer im selben Reaktionsansatz ablaufender enzymatisch katalysierter Redoxreaktionen einer der beiden Redoxkofaktoren in seiner reduzierten Form anfällt und der jeweils andere in seiner oxidierten Form, und
B) D-Fructose zu Furanderivaten umgesetzt wird und
in Stufe A)
- bei der Regenerierungsreaktion, die den reduzierten Kofaktor wieder in seine ursprüngliche oxidierte Form überführt, Sauerstoff oder eine Verbindung der allgemeinen Formel 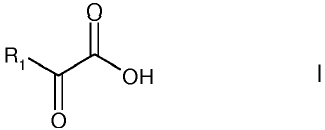 worin R₁ für eine geradkettige oder verzweigtkettige (C₁-C₄)-Alkylgruppe oder für eine (C₁-C₄)-Carboxyalkylgruppe steht, reduziert wird, und
- bei der Regenerierungsreaktion, die den oxidierten Kofaktor wieder in seine ursprüngliche reduzierte Form überführt, ein (C₄-C₈)-Cycloalkanol oder eine Verbindung der allgemeinen Formel 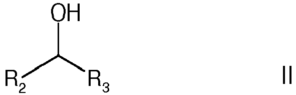 worin R₂ und R₃ unabhängig voneinander ausgewählt sind aus der Gruppe bestehend aus H, (C₁-C₆)-Alkyl, worin Alkyl geradkettig oder verzweigt ist, (C₁-C₆)-Alkenyl, worin Alkenyl geradkettig oder verzweigt ist und ein bis drei Doppelbindungen enthält, Aryl, insbesondere C₆-C₁₂ Aryl, Carboxyl, oder (C₁-C₄)-Carboxyalkyl, insbesondere auch Cycloalkyl, insbesondere C₃-C₈ Cycloalkyl, oxidiert wird.

2. Verfahren nach Anspruch 1, **dadurch gekennzeichnet, dass** an der Umwandlung von D-Glucose zu D-Fructose mindestens eine Dehydrogenase eingesetzt wird.

3. Verfahren nach Anspruch 2, **dadurch gekennzeichnet, dass** als Redoxkofaktor(en) NAD⁺/NADH und NADP⁺/NADPH, entweder in löslicher Form oder an Feststoffe immobilisiert, eingesetzt werden.

4. Verfahren nach einem der Ansprüche 1 bis 3, **dadurch gekennzeichnet, dass** mindestens ein Redoxkofaktor bei der Umsetzung von D-Glucose zu D-Fructose im selben Reaktionsansatz durch mindestens ein weiteres Redoxenzym, ausgewählt aus Alkoholdehydrogenasen, NADH-Oxidasen, Hydrogenasen, Laktatdehydrogenasen oder Formiatdehydrogenasen unter Verbrauch von Co-Substraten, regeneriert wird.

5. Verfahren nach Anspruch 4, **dadurch gekennzeichnet, dass** Co-Substrate ausgewählt sind aus Alkoholen, Milchsäure und deren Salze, Brenztraubensäure und deren Salze, Sauerstoff, Wasserstoff und/oder Ameisensäure und deren Salze

6. Verfahren nach einem der Ansprüche 1 bis 5, **dadurch gekennzeichnet, dass** die Reaktion in Stufe a) nach dem Reaktionsschema 3 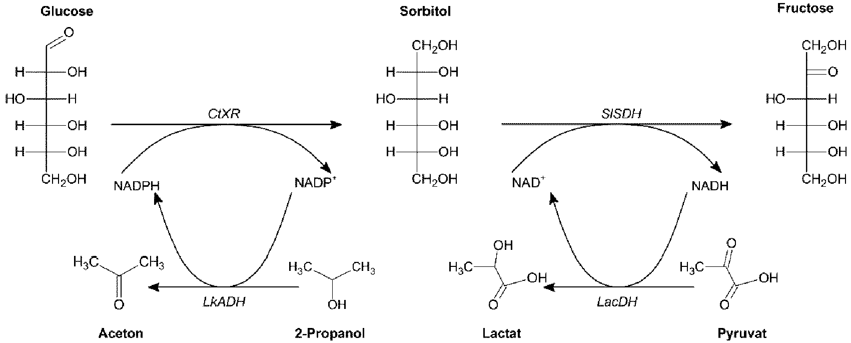 worin
CtXR = Xylosereduktase *aus Candida tropicalis*
SISDH = Sorbitoldehydrogenase aus Schafsleber
LkADH = Alkoholdehydrogenase aus *Lactobacillus kefir,* NADP(H)-abhängig
LacDH = Laktatdehydrogenase, NAD(H)-abhängig
bedeutet,
oder nach dem Reaktionsschema 4 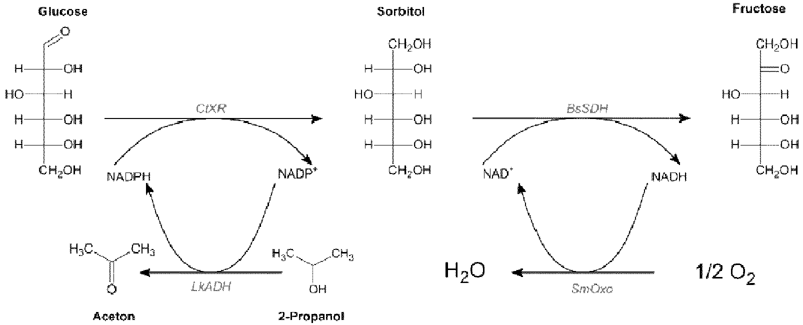 worin
CtXR = Xylosereduktaseaus *Candida tropicalis*
BsSDH = Sorbitol-Dehydrogenase aus *Bacillus subtilis*
LkADH = *kefir* Alkoholdehydrogenase aus *Lactobacillus* NADP(H)-abhängig
SmOxo = NADH Oxidase aus *Streptococcus mutans*
bedeutet,
abläuft.

7. Verfahren gemäß einem der vorhergehenden Ansprüche, **dadurch gekennzeichnet, dass** die D-Fructose, die gemäß Stufe A) der vorliegender Erfindung gewonnen wird, isoliert wird.

8. Verfahren gemäß Anspruch 7 **dadurch gekennzeichnet, dass** die D-Fructose in kristallisierter Form isoliert wird.

9. Verfahren gemäß einem der vorhergehenden Ansprüche, **dadurch gekennzeichnet, dass** in Stufe B) ein saurer Katalysator und ein Lösungsmittel eingesetzt werden.

10. Verfahren nach Anspruch 9, **dadurch gekennzeichnet, dass** N-Methyl-2-pyrrolidon der Formel 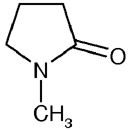 als Lösungsmittel eingesetzt wird.

11. Verfahren nach Anspruch 10, **dadurch gekennzeichnet, dass** N-Methyl-2-pyrrolidon entweder als Reaktions-Lösungsmittel oder als Co-Lösungsmittel verwendet wird.

12. Verfahren gemäß einem der vorhergehenden Ansprüche, **dadurch gekennzeichnet, dass** die Umwandlung von D-Fructose zu Furanderivaten in Stufe B) als Batch-Prozess oder als kontinuierlicher Prozess durchgeführt wird.

13. Verfahren gemäß Anspruch 12, **dadurch gekennzeichnet, dass** die Umwandlung von D-Fructose zu Furanderivaten in Stufe B) unter Mikrowellen-Erhitzung durchgeführt wird.

14. Verfahren nach einem der Ansprüche 9 bis 13, **dadurch gekennzeichnet, dass** bei der Umsetzung von D-Fructose zu Furanderivaten in Stufe B) als saurer Katalysator
- ein homogener Säurekatalysator;
- ein heterogener Säurekatalysator,
- ein Lewis-Säurekatalysator,
- ein SILP-Katalysator,
eingesetzt wird.

15. Verfahren nach Anspruch 14, **dadurch gekennzeichnet, dass** als homogener Säurekatalysator Schwefelsäure oder Salzsäure eingesetzt wird.

16. Verfahren nach Anspruch 14, **dadurch gekennzeichnet, dass** als heterogener Säurekatalysator ein Ionenaustauscher, insbesondere ein Montmorillonit oder ein Amberlit eingesetzt wird.

17. Verfahren nach Anspruch 14, **dadurch gekennzeichnet, dass** als Lewis-Säurekatalysator CrCl₂, AlCl₃ oder SiO₂-MgCl₂ eingesetzt wird.

18. Verfahren gemäß einem der vorhergehenden Ansprüche, **dadurch gekennzeichnet, dass** das Furanderivat Hydroxymethylfurfural der Formel 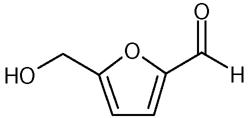 ist.

19. Verfahren gemäß einem der vorhergehenden Ansprüche, **dadurch gekennzeichnet, dass** hergestellte Furanderivate weiter umgesetzt werden.

20. Verfahren gemäß Anspruch 19, **dadurch gekennzeichnet, dass** Hydroxymethylfurfural weiter zu 2,5-Furandicarbonsäure (FDCA) der Formel 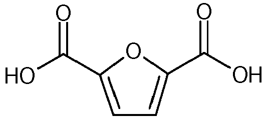 oxidiert wird, das gegebenenfalls einer Polymerisation unterworfen wird, insbesondere zur Herstellung von Polyethylenfuranoat.

## Claims

1. A process for the production of furan derivatives from D-glucose, **characterized in that**
A) D-glucose is converted into D-fructose in an enzymatic process, wherein redox cofactors are used and regenerated,
wherein D-glucose is converted into D-fructose, involving two or more oxidoreductases in product-forming reactions, and, during the conversion of D-glucose into D-fructose, at first an enzymatically catalyzed reduction to D-sorbitol and, subsequently, an enzymatically catalyzed oxidation of D-sorbitol to D-fructose are performed,
wherein NAD⁺/NADH and NADP⁺/NADPH are used as redox cofactors and, as a result of at least two further enzymatically catalyzed redox reactions proceeding in the same reaction batch, one of the two redox cofactors accumulates in its reduced form and, respectively, the other one in its oxidized form, and
B) D-fructose is converted into furan derivatives, and
in stage A)
- in the regeneration reaction reconverting the reduced cofactor into its original oxidized form, oxygen or a compound of general formula 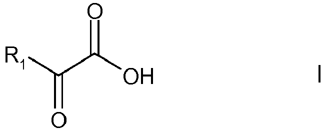 wherein R₁ represents a linear-chain or branched (C₁-C₄)-alkyl group or a (C₁-C₄)-carboxyalkyl group, is reduced, and
- in the regeneration reaction reconverting the oxidized cofactor into its original reduced form, a (C₄-C₈)-cycloalkanol or a compound of general formula 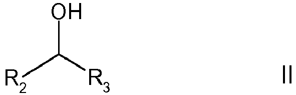
wherein R₂ and R₃ are independently selected from the group consisting of H, (C₁-C₆)-alkyl, wherein alkyl is linear-chain or branched, (C₁-C₆)-alkenyl, wherein alkenyl is linear-chain or branched and contains one to three double bonds, aryl, in particular C₆-C₁₂-aryl, carboxyl, or (C₁-C₄)-carboxyalkyl, in particular also cycloalkyl, in particular C₃-C₈-cycloalkyl, is oxidized.

2. A process according to claim 1, **characterized in that** at least one dehydrogenase is used for the conversion of D-glucose into D-fructose.

3. A process according to claim 2, **characterized in that** NAD⁺/NADH and NADP⁺/NADPH, either in a soluble form or immobilized onto solids, are used as redox cofactor(s).

4. A process according to any one of claims 1 to 3, **characterized in that** at least one redox cofactor is regenerated during the conversion of D-glucose into D-fructose in the same reaction batch by at least one further redox enzyme selected from alcohol dehydrogenases, NADH oxidases, hydrogenases, lactate dehydrogenases or formate dehydrogenases, under consumption of co-substrates.

5. A process according to claim 4, **characterized in that** co-substrates are selected from alcohols, lactic acid and salts thereof, pyruvic acid and salts thereof, oxygen, hydrogen and/or formic acid and salts thereof.

6. A process according to any one of claims 1 to 5, **characterized in that** the reaction in stage a) proceeds according to Reaction Scheme 3 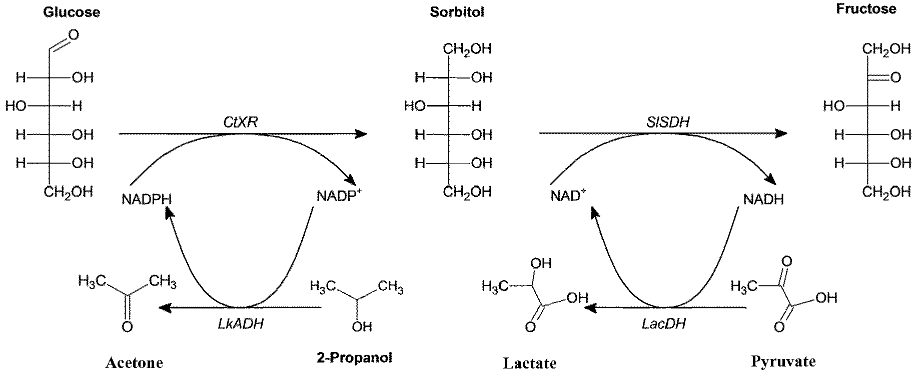 wherein
CtXR = xylose reductase from *Candida tropicalis*
SISDH = sorbitol dehydrogenase from sheep liver
LkADH = alcohol dehydrogenase from *Lactobacillus kefir*, NADP(H)-dependent
LacDH = lactate dehydrogenase, NAD(H)-dependent
or according to Reaction Scheme 4 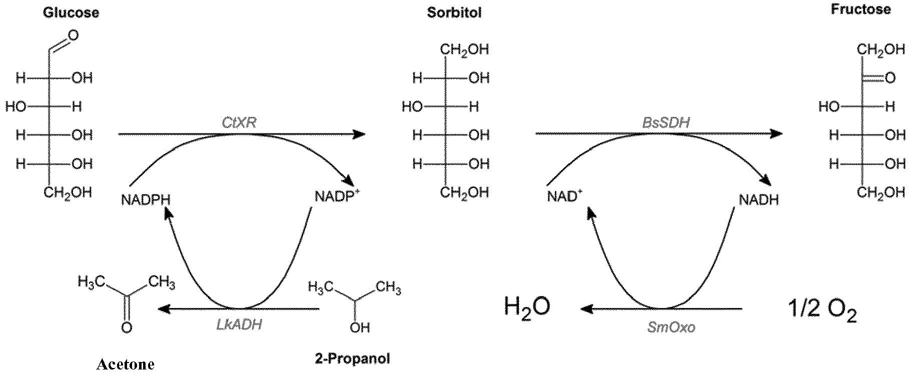 wherein
CtXR = xylose reductase from *Candida tropicalis*
BsSDH = sorbitol dehydrogenase from *Bacillus subtilis*
LkADH = alcohol dehydrogenase from *Lactobacillus kefir*, NADP(H)-dependent
SmOxo = NADH oxidase from *Streptococcus mutans.*

7. A process according to any one of the preceding claims, **characterized in that** the D-fructose obtained according to stage A) of the present invention is isolated.

8. A process according to claim 7, **characterized in that** the D-fructose is isolated in a crystalline form.

9. A process according to any one of the preceding claims, **characterized in that** an acidic catalyst and a solvent are used in stage B).

10. A process according to claim 9, **characterized in that** N-methyl-2-pyrrolidone of formula 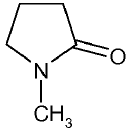 is used as a solvent.

11. A process according to claim 10, **characterized in that** N-methyl-2-pyrrolidone is used either as a reaction solvent or as a co-solvent.

12. A process according to any one of the preceding claims, **characterized in that** the conversion of D-fructose into furan derivatives in stage B) is performed as a batch process or as a continuous process.

13. A process according to claim 12, **characterized in that** the conversion of D-fructose into furan derivatives in stage B) is performed under microwave heating.

14. A process according to any one of claims 9 to 13, **characterized in that**
- a homogeneous acid catalyst,
- a heterogeneous acid catalyst,
- a Lewis acid catalyst,
- an SILP catalyst,
is used as the acidic catalyst during the conversion of D-fructose into furan derivatives in stage B).

15. A process according to claim 14, **characterized in that** sulphuric acid or hydrochloric acid is used as the homogeneous acid catalyst.

16. A process according to claim 14, **characterized in that** an ion exchanger, in particular a montmorillonite or an Amberlite, is used as the heterogeneous acid catalyst.

17. A process according to claim 14, **characterized in that** CrCl₂, AlCl₃ or SiO₂-MgCl₂ is used as the Lewis acid catalyst.

18. A process according to any one of the preceding claims, **characterized in that** the furan derivative is hydroxymethylfurfural of formula

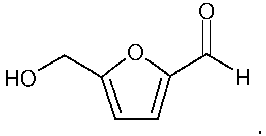

19. A process according to any one of the preceding claims, **characterized in that** furan derivatives which are produced are converted further.

20. A process according to claim 19, **characterized in that** hydroxymethylfurfural is oxidized further into 2,5-furan dicarboxylic acid (FDCA) of formula 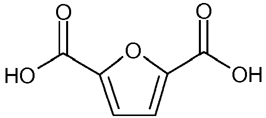 which, optionally, is subjected to polymerization, in particular for the production of polyethylene furanoate.

## Revendications

1. Procédé pour la production de dérivés de furane à partir du D-glucose, **caractérisé en ce que**
A) on transforme le D-glucose en D-fructose dans un procédé enzymatique avec utilisation et régénération de cofacteurs redox,
dans lequel on transforme le D-glucose en D-fructose avec participation de deux ou plusieurs oxydoréductases dans des réactions de formation de produit et lors de la conversion de D-glucose en D-fructose, on effectue d'abord une réduction en D-sorbitol avec catalyseur enzymatique et ensuite une oxydation avec catalyseur enzymatique de D-sorbitol en D-fructose,
dans lequel on utilise comme cofacteurs redox NAD⁺/NADH et NADP⁺/NADPH et on obtient comme résultat d'au moins deux autres réactions redox avec catalyseur enzymatique se déroulant dans le même mélange de réaction un des deux cofacteurs redox dans sa forme réduite et respectivement l'autre dans sa forme oxydée, et
B) on convertit le D-fructose en dérivés de furane et
dans l'étape A)
- lors de la réaction de régénération, qui transforme le cofacteur réduit de nouveau dans sa forme oxydée initiale, on réduit l'oxygène ou un composé de la formule générale 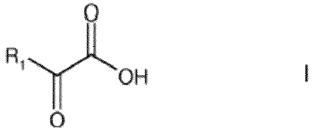 dans laquelle R₁ représente un groupe alkyle (C₁-C₄) à chaîne droite ou à chaîne ramifiée ou un groupe carboxyalkyle (C₁-C₄), et
- lors de la réaction de régénération, qui transforme le cofacteur oxydé de nouveau dans sa forme réduite initiale, on oxyde un cycloalcanol (C₄-C₈) ou un composé de la formule générale 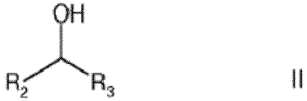
dans laquelle R₂ et R₃ sont choisis indépendamment l'un de l'autre dans le groupe composé de H, alkyle (C₁-C₆) dans lequel l'alkyle est à chaîne droite ou ramifiée, alcényle (C₁-C₆), dans lequel l'alcényle est à chaîne droite ou ramifiée et contient une à trois liaisons doubles, aryle, en particulier aryle (C₆-C₁₂), carboxyle, ou carboxyalkyle (C₁-C₄), en particulier aussi cycloalkyle, en particulier cycloalkyle (C₃-C₈).

2. Procédé selon la revendication 1, **caractérisé en ce que** l'on utilise au moins une déshydrogénase lors de la conversion de D-glucose en D-fructose.

3. Procédé selon la revendication 2, **caractérisé en ce que** l'on utilise comme cofacteur(s) redox NAD⁺/NADH et NADP⁺/NADPH, soit sous forme soluble soit sous forme immobilisée sur des substances solides.

4. Procédé selon l'une quelconque des revendications 1 à 3, **caractérisé en ce que** l'on régénère au moins un cofacteur redox lors de la conversion de D-glucose en D-fructose dans le même mélange de réaction au moyen d'au moins une enzyme redox, choisie parmi les alcools déshydrogénases, les NADH-oxydases, les hydrogénases, les lactates déshydrogénases ou les formiates déshydrogénases avec consommation de co-substrats.

5. Procédé selon la revendication 4, **caractérisé en ce que** les co-substrats sont choisis parmi les alcools, l'acide lactique et ses sels, l'acide pyruvique et ses sels, l'oxygène, l'hydrogène et/ou l'acide formique et ses sels.

6. Procédé selon l'une quelconque des revendications 1 à 5, **caractérisé en ce que** la réaction à l'étape a) se déroule selon le schéma de réaction 3 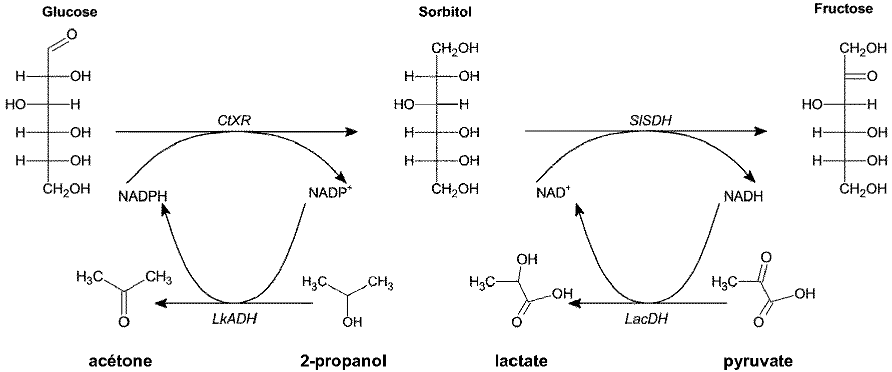 dans lequel on a
CtXR = xylose réductase de *Candida tropicalis*
SISDH = sorbitol déshydrogénase de foie d'ovin
LkADH = alcool déshydrogénase de *Lactobacillus kefir,* dépendant de NADP(H)
LacDH = lactate déshydrogénase, dépendant de NAD(H),
ou selon le schéma de réaction 4 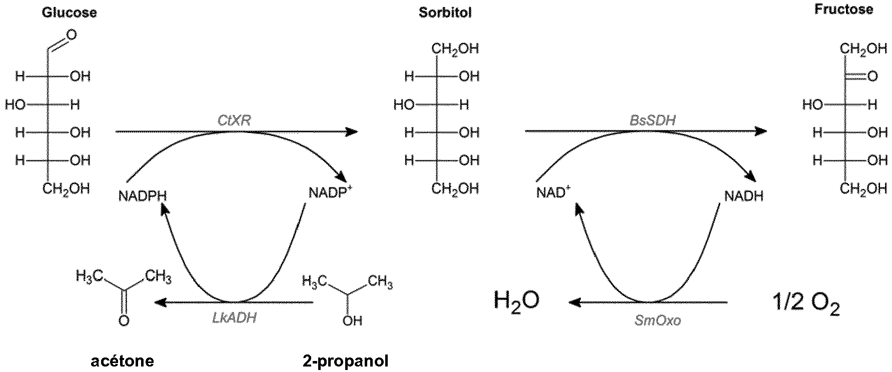 dans lequel on a
CtXR = xylose réductase de *Candida tropicalis*
BsSDH = sorbitol déshydrogénase de *Bacillus subtilis*
LkADH = alcool déshydrogénase de *Lactobacillus kefir,* dépendant de NADP(H)
SmOxo = NADH oxydase de *Streptococcus mutans.*

7. Procédé selon l'une quelconque des revendications précédentes, **caractérisé en ce que** l'on isole le D-fructose, qui a été produit à l'étape A) de la présente invention.

8. Procédé selon la revendication 7, **caractérisé en ce que** l'on isole le D-fructose sous forme cristallisée.

9. Procédé selon l'une quelconque des revendications précédentes, **caractérisé en ce que** l'on utilise à l'étape B) un catalyseur acide et un solvant.

10. Procédé selon la revendication 9, **caractérisé en ce que** l'on utilise comme solvant le N-méthyl-2-pyrrolidone de la formule

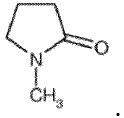

11. Procédé selon la revendication 10, **caractérisé en ce que** l'on utilise le N-méthyl-2-pyrrolidone soit comme solvant de réaction soit comme co-solvant.

12. Procédé selon l'une quelconque des revendications précédentes, **caractérisé en ce que** l'on effectue la conversion de D-fructose en dérivés de furane à l'étape B) dans un processus par charges ou dans un processus continu.

13. Procédé selon la revendication 12, **caractérisé en ce que** l'on effectue la conversion de D-fructose en dérivés de furane à l'étape B) sous chauffage par microondes.

14. Procédé selon l'une quelconque des revendications 9 à 13, **caractérisé en ce que**, lors de la conversion de D-fructose en dérivés de furane à l'étape B), on utilise comme catalyseur acide
- un catalyseur acide homogène,
- un catalyseur acide hétérogène,
- un catalyseur acide de Lewis,
- un catalyseur SILP.

15. Procédé selon la revendication 14, **caractérisé en ce que** l'on utilise comme catalyseur acide homogène l'acide sulfurique ou l'acide chlorhydrique.

16. Procédé selon la revendication 14, **caractérisé en ce que** l'on utilise comme catalyseur acide hétérogène un échangeur d'ions, en particulier une montmorillonite ou une amberlite.

17. Procédé selon la revendication 14, **caractérisé en ce que** l'on utilise comme catalyseur acide de Lewis du CrCl₂, du AlCl₃ ou du SiO₂-MgCl₂.

18. Procédé selon l'une quelconque des revendications précédentes, **caractérisé en ce que** le dérivé de furane est l'hydroxyméthylfurfural de la formule

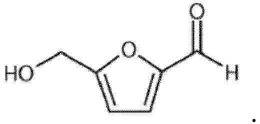

19. Procédé selon l'une quelconque des revendications précédentes, **caractérisé en ce que** l'on convertit de nouveau les dérivés de furane produits.

20. Procédé selon la revendication 19, **caractérisé en ce que** l'on oxyde ensuite l'hydroxyméthylfurfural en acide 2,5-furandicarboxylique (FDCA) de la formule 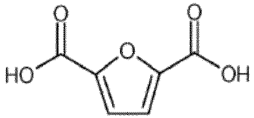 que l'on soumet éventuellement à une polymérisation, en particulier pour la production de polyéthylène furanoate.
